# EUROPEAN PATENT APPLICATION

(11) **EP 4 516 292 A1**
(43) Date of publication of application: **05.03.2025**
(21) Application number: 23795441.7
(22) Date of filing: 26.04.2023
(51) Int. Cl.: A61K 9/19, A61K 47/42, A61K 31/337, A61P 35/00

(54) **INSTANT NANOPARTICLE COMPOSITION AND PREPARATION METHOD THEREFOR**

(30) Priority: 27.04.2022 CN 202210450810
(71) Applicant: CSPC Zhongqi Pharmaceutical Technology (Shijiazhuang) Co., Ltd., Shijiazhuang, Hebei 050035 (CN)
(72) Inventor: LI, Chunlei, Shijiazhuang, Hebei 050035 (CN); LI, Yanhui, Shijiazhuang, Hebei 050035 (CN); LI, Mengmeng, Shijiazhuang, Hebei 050035 (CN); LI, Yongfeng, Shijiazhuang, Hebei 050035 (CN); LI, Jingjing, Shijiazhuang, Hebei 050035 (CN); WANG, Caixia, Shijiazhuang, Hebei 050035 (CN); WANG, Danqing, Shijiazhuang, Hebei 050035 (CN); TIAN, Haiwei, Shijiazhuang, Hebei 050035 (CN); WANG, Shixia, Shijiazhuang, Hebei 050035 (CN); WANG, Yeming, Shijiazhuang, Hebei 050035 (CN)
(74) Representative: Barrow, Nicholas Martin
(86) International application number: PCT/CN2023/090721
(87) International publication number: WO 2023/208009

(57) **Abstract**

A nanoparticle composition for rapid suspension and a preparation method therefor. The composition comprises an active ingredient, albumin and a particle stabilizer, and optionally a lyoprotectant. The active ingredient has the following characteristics: insoluble or slightly soluble in water, and soluble or easily soluble in specific organic solvents, and is preferably paclitaxel or docetaxel.

## Description

### TECHNICAL FIELD

The present invention relates to the field of pharmaceuticals, and more specifically to a nanoparticle composition for rapid suspension comprising human serum albumin and a hydrophobic drug and a preparation method therefor.

### BACKGROUND

Paclitaxel, which is an anti-cancer drug extracted from the trunk and bark of the Taxus plants, is a mitotic microtubule inhibitor that has the effect of polymerizing and stabilizing intracellular microtubules. During the mitosis stage, paclitaxel prevents microtubules from separating, thereby blocking cells in the G2 and M phases of the cell cycle. Therefore, paclitaxel causes rapidly dividing tumor cells to be fixed in the mitotic stage, such that the cell replication is blocked and the cells die. Paclitaxel has important clinical activity against various cancers (such as breast cancer, ovarian cancer, lung cancer, bladder cancer, etc.).

However, there is difficulty in administration of paclitaxel in human due to its poor water solubility. To make paclitaxel suitable for intravenous injection, Bristol-Myers Squibb (BMS) developed Taxol^{®} in which the surfactant polyoxyethylene castor oil (Cremophor^{®} EL) and absolute ethanol are used together as the solvent to increase the solubility of paclitaxel. Taxol has significant activity against ovarian cancer, breast cancer, lung cancer, esophageal cancer, and head and neck cancer. However, Taxol has been shown to induce administration-related toxicities, as well as significant acute and cumulative toxicities such as myelosuppression, neutropenic fever, anaphylaxis, etc. These side effects are related to the used surfactant, polyoxyethylene castor oil (Anantbhushan et al., Asia Pac J Clin Oncol. 2013;9:176-181). According to clinical study reports and post-marketing safety data, the overall incidence of allergic reactions to Taxol is approximately 39%. Currently, patients need to be given antihistamines and steroids in advance to attenuate the side effects caused by the surfactant when using Taxol.

US5439686, US6537579, US6749868, US2006121119, CN97199720.9, CN03108361.7 and CN200610077006.4 disclose a method for preparing paclitaxel albumin nanoparticles, obtaining a formulation with significant advantages and developing the marketed product Abraxane^{®}, wherein the advantages include 1) no pretreatment with antihistamines before administration, the significantly reduced incidence of allergic reactions than Taxol^{®}, and no influence on the activity of PD-1/PDL1 when used in combination with PD-1/PDL1; 2) shortened infusion time from 3 hours for ordinary preparations to 30 minutes; 3) improved safety and effectiveness because it is rapidly disintegrated and disperses after injection into the blood and transported to the tumor tissues through a special transport mechanism.

However, this system still has many shortcomings, for example, the preparation before using the lyophilized powder injection of albumin-bound paclitaxel, Abraxane^{®}, is complicated and includes six steps: 1) under aseptic operation, use 20 ml of 0.9% sodium chloride injection per vial to disperse and dissolve; 2) slowly inject the 20 ml of 0.9% sodium chloride injection along the inner wall of the vial with a sterile syringe, with the injection time of not less than 1 minute; 3) do not inject the 0.9% sodium chloride injection directly onto the lyophilized cake/powder to avoid the formation of foam; 4) after injection, allow the vial to stand for at least 5 minutes to ensure that the lyophilized cake/powder is completely soaked; 5) gently shake the vial or slowly turn the vial upside down for at least 2 minutes to allow all lyophilized cakes/powder in the vial to be completely dispersed and dissolved to avoid the formation of foam; 6) if foam occurs, leave it still for 15 minutes until the foam subsides. According to this standard preparation operation, it takes a minimum of 8 minutes, and if foam is accidentally generated, it will take more than 23 minutes. This preparation operation before clinical use is extremely complicated, and it is easy for the preparation personnel to operate in an irregular way; the product dispersion time is long, and the risk of contamination increases during drug preparation; the preparation operation takes up a lot of space and time, affecting the work efficiency of the preparation personnel; and when the preparation operation is irregular, it is easy to generate a lot of foam and occur incomplete dissolution, which may lead to problems such as inaccurate liquid metering and clogging of infusion lines, and may affect medication safety or lead to medical disputes.

In addition, this system comprises a lot of human serum albumin (HSA) that may cause allergies. The source of HSA is still human blood. The safety of blood products is at risk due to possible contamination during blood collection and storage. Additionally, HSA is expensive and still in short supply in some areas.

CN201580045838.5 filed by the present applicant in 2015 provides purified therapeutic nanoparticles consisting of an active ingredient and human serum albumin, wherein the weight ratio of human serum albumin to the active ingredient is selected from a range from 0.01:1 to 8.5:1, and wherein the therapeutic nanoparticles are substantially free of free HAS that is not comprised in the nanoparticles. In some embodiments, when the pharmaceutical composition is provided in the form of a lyophilized powder, it comprises one or more excipients for lyophilization selected from the group consisting of mannitol, sucrose, lactose, maltose, trehalose, dextran, or a combination thereof. The therapeutic nanoparticles disclosed in this patent are intended to be administered via intravenous infusion, so the sterility of the product needs to be ensured. Both the nanoparticles and human serum albumin are temperature sensitive, so they cannot be sterilized by heat sterilization, and possible sterilization solutions include full aseptic production or sterilization by filtration. In this case, the suspension obtained after removing the organic solvent is sterilized by filtration through a filter membrane, and then lyophilized to obtain a solid, which is resuspended in water. In some embodiments, the solid is resuspended in water and the paclitaxel is adjusted to about 5 mg/ml.

Ismael B et al. (Reversible exposure of hydrophobic residues on albumin as a novel strategy for formulation of nanodelivery vehicles for taxanes[J]. International Journal of Nanomedicine, 2011, 2011:1193-1200.) reported in 2011 a method of enhancing the solubility of paclitaxel by simply incubating a paclitaxel solution with a HSA solution without using emulsification and homogenization. In this method, HSA needs to be pretreated (i.e. adjusting the pH of the HSA solution to 2.7 to expose the hydrophobic end of the protein to enhance its binding ability to paclitaxel), and then the paclitaxel dissolved in an organic solvent is slowly added and the pH is adjusted to 7.0. NaCl (0, 0.2M, 0.4M, 0.6M, 0.8M and 1.0M were examined) was added during the preparation, and it was shown that when NaCl was 0-0.8M (corresponding to a weight percentage of 0-4.6%), it had a solubilizing effect on paclitaxel, and when NaCl was 0.4M (corresponding to a weight percentage of 2.3%), paclitaxel and HSA had the highest binding rate of 91.0%. In this method, the added NaCl was not removed from the system. This report does not mention whether the system comprised nanoparticles, nor did it involve lyophilizing the system, let alone studying issues that urgently need to be addressed in the prior art such as the stability before and after lyophilization and the reconstitution time.

### SUMMARY OF THE INVENTION

The composition of the present invention shortens the reconstitution time of the preparation to be within 2 minutes, and can reduce foaming and avoid problems such as incomplete dissolution. Moreover, the composition of the present invention can maintain the functional structure and stability of the nanoparticles unchanged, and has the same safety and therapeutic effect. Furthermore, in the nanoparticle composition for rapid suspension of the present invention, the amount of HSA is reduced and thus allergic reactions caused by HSA can be reduced.

Compared with the research of Ismael B et al. in 2011, the preparation process of the present invention involves the formation of nanoparticles and does not need adjustment of the pH of the system. Surprisingly, the present inventors found that it is necessary not only to adopt an appropriate concentration of NaCl during preparation of the nanoparticle composition for rapid suspension, but also to control the NaCl content in the finally obtained nanoparticle composition for rapid suspension.

### Terminology:

The term "nanoparticles" as used herein refers to particles having a nanoscale size, e.g., a size of about 1 nm, about 10 nm or about 100 nm, in at least one dimension, e.g., one, two or three dimensions.

The term "purified nanoparticles" as used herein refers to nanoparticles formed from human serum albumin and an active ingredient, in which the free human serum albumin has been removed to 5% to 50% of the original amount.

The term "nanoparticle composition for rapid suspension" used herein refers to a nanoparticle composition which is obtained by lyophilizing the above-mentioned purified nanoparticles and can be rapidly dispersed upon addition of a solvent.

The term "about" as used herein means greater than or less than 10% of the specified value. For example, "about 50nm" means 45nm to 55nm.

"Human serum albumin monomer" or "HSA monomer" refers to a soluble globulin composed of 585 amino acids, with a molecular weight of about 66,000 Daltons. It is the most abundant protein in human plasma and the last peak in size exclusion chromatography, and accounts for the vast majority of normal human serum albumin products. HSA has multiple hydrophobic binding sites and can bind a set of diverse drugs, particularly neutral or negatively charged hydrophobic compounds.

The term "active ingredient" as used herein means a pharmaceutically active ingredient. In particular, the active ingredient means any substance or entity capable of exerting a therapeutic effect, e.g., treating, preventing, alleviating or inhibiting any disease and/or condition.

The term "dialysis fold" refers to the ratio of the volume of dialysate consumed to the volume of feed after dialysis when the volume of the feed does not change during isovolumetric dialysis.

The term "lyoprotectant" refers to a substance added to the pharmaceutical composition comprising the purified nanoparticles in order to protect the nanoparticles during lyophilization.

The term "treatment" or "therapy" refers to a clinical regimen for a disease, disorder, or health condition in a patient (see, for example, Stedman's Medical Dictionary), and "treating a tumor" means reducing the number of symptoms of a tumor, reducing the severity of one or more symptoms of a tumor, or slowing the progression of a tumor.

A "therapeutically effective amount" of a particular therapeutic agent is an amount of the therapeutic agent sufficient to reduce the number of tumor symptoms, reduce the severity of one or more tumor symptoms, or slow the progression of the tumor.

In a first aspect, the present invention provides a nanoparticle composition for rapid suspension comprising an active ingredient, an albumin, a particle stabilizer, and optionally a lyoprotectant.

In some embodiments, the present invention provides a nanoparticle composition for rapid suspension comprising an active ingredient, an albumin, a lyoprotectant and a particle stabilizer.

The active ingredient is selected from active ingredients suitable for being wrapped in human serum albumin, and should have the following characteristics: insoluble or slightly soluble in water, and soluble or easily soluble in specific organic solvents.

In some embodiments, the active ingredient is selected from the group consisting of taxanes, including but not limited to paclitaxel, docetaxel, cabazitaxel, or lipophilic derivatives of docetaxel; macrolides, including but not limited to rapamycin and derivatives thereof, epothilone B and derivatives thereof, tanespimycin and derivatives thereof, etc.; camptothecins, including but not limited to 10-hydroxycamptothecin, SN38 and derivatives thereof, etc.; anthracyclines, including but not limited to aclarubicin, pirarubicin, etc.; and other active ingredients, including colchicine and derivatives thereof, thiocolchicine dimer, amiodarone, liothyronine, cyclosporine, exemestane, flutamide, fulvestrant, romidepsin, semustine, ibuprofen, etc.

In some embodiments, the active ingredient is selected from taxanes, preferably, the taxane is selected from the group consisting of paclitaxel or docetaxel.

The albumin is selected from serum albumin with carrier function, such as human serum albumin and bovine serum albumin, preferably human serum albumin.

The lyoprotectant is selected from the group consisting of mannitol, sucrose, lactose, maltose, trehalose, dextran or any combination thereof, preferably a combination of mannitol and sucrose, and the ratio therebetween is preferably 10:1 to 1:1, preferably 8:1 to 2:1, preferably 8:1 to 3:1, further preferably 5:1 to 2:1, further preferably 5:1 to 3:1, and most preferably 5:1.

The particle stabilizer is selected from the group consisting of sodium chloride, disodium hydrogen phosphate, sodium dihydrogen phosphate, and potassium chloride, preferably sodium chloride.

In some embodiments, the nanoparticle composition for rapid suspension comprises, by weight percentage, 1-30% of an active ingredient, 0.1-30% of a protein, 0.00001-3% of a particle stabilizer, and balance of lyoprotectant.

In some embodiments, the nanoparticle composition for rapid suspension comprises, by weight percentage, 0.00001-3%, preferably 0.0001-1%, or any subrange within the range, for example, 0.0001-1%, 0.0001-0.1%, 0.0005-0.5%, 0.001-0.1%, 0.005-0.05%, 0.008-0.022% of the particle stabilizer.

In some embodiments, the nanoparticle composition for rapid suspension comprises, by weight percentage, 1-30%, preferably 2-20% or 3-15% or 5-10% of the active ingredient.

In some embodiments, the nanoparticle composition for rapid suspension comprises, by weight percentage, 0.1-30% or 1-25% or 5-20% of the protein.

In some embodiments, the binding rate of the active ingredient to protein is great than 90%.

In the composition, the weight ratio of the protein to the active ingredient is 0.1:1-5:1, preferably 0.3:1-3:1 or 0.5:1-3:1 or 1:1-3:1 or 0.5:1-2.5:1, for example, is selected from the group consisting of 0.1:1, 0.2:1, 0.3:1, 0.4:1, 0.5:1, 0.6:1, 0.70:1, 0.8:1, 0.9:1, 0.95:1, 1:1, 1.1:1 , 1.2:1, 1.3:1, 1.4:1, 1.5:1, 1.6:1, 1.7:1, 1.8:1, 1.8:1, 1.9:1, 2:1, 2.1:1, 2.2:1, 2.3 :1, 2.4:1, 2.5:1, 2.6:1, 2.7:1, 2.8:1, 2.9:1, 3:1, 3.5:1, 4:1, 4.5:1, 5:1 or a range between any two of the above ratios. In some specific embodiments, the protein is human serum albumin.

In some embodiments, the nanoparticle for rapid suspension in the composition have an average particle size of 30-200 nm, preferably 100-180 nm, preferably 110-170 nm, more preferably 110-160 nm, more preferably 110-140 nm, further preferably 115-130 nm, more preferably 118-125 nm, most preferably 118-122 nm, for example, selected from the group consisting of 30, 40, 50, 60, 70, 80, 90, 100, 110, 111, 112, 113, 114, 115, 116, 117, 118, 119, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 165, 170, 175, 180, 185, 190, 195, 200 nm or a range between any two of the above values.

In some embodiments, an organic solvent is used to dissolve the active ingredient. A skilled person can select an appropriate organic solvent based on the nature of the active ingredient. The organic solvent is selected from the group consisting of pure solvents with low water solubility and low boiling point or a mixed solvent thereof with a small molecular alcohol. Preferably, the organic solvent is one or more selected from the group consisting of chloroform, dichloromethane, ethanol or tert-butyl alcohol, preferably chloroform or a combination of chloroform and ethanol. In the present invention, the organic solvent can be removed from the emulsion by any appropriate method, such as evaporation under reduced pressure, dialysis etc., to minimize the residual organic solvent in the product. In the nanoparticle composition for rapid suspension, the residual amount of the organic solvent is less than 0.1 mg/ml, preferably less than 0.08 mg/ml, 0.05 mg/ml, 0.04 mg/ml, 0.03 mg/ml, 0.02 mg/ml or 0.01 mg/ml, further preferably less than 5 mg/ml, 1 mg/ml, 0.5 mg/ml, 0.1 mg/ml, 0.05 mg/ml or 0.01 µg/ml.

In some embodiments, the nanoparticle composition for rapid suspension does not comprise any surfactant, such as polyoxyethylene castor oil, polyvinylpyrrolidone, polyvinyl alcohol, hydroxypropyl methylcellulose, poloxamer, lecithin, cholates, etc.

In a second aspect, the present invention provides a pharmaceutical composition comprising the nanoparticle composition for rapid suspension of the first aspect.

In some embodiments, the pharmaceutical composition is in a solid or liquid form.

In some embodiments, the pharmaceutical composition is provided in a liquid form, including, but not limited to, a form suitable for injection to a subject. In a specific embodiment, the pharmaceutical composition is an injection solution.

In some specific embodiments, the pharmaceutical composition is provided in a liquid form, wherein the nanoparticle for rapid suspension are suspended in a pharmaceutically acceptable carrier. The pharmaceutically acceptable carrier includes, but not limited to, a buffer, a preservative, water for injection, physiological saline, and isotonic solution. In some specific embodiments, the liquid form pharmaceutical composition of the present invention comprises the active ingredient (such as paclitaxel) in a content of 0.1-100 mg/ml, preferably 0.5-50 mg/ml, more preferably 1-20 mg/ml, such as 5 mg/ml.

In other embodiments, the pharmaceutical composition is in a solid form, including but not limited to dry powder or lyophilized powder.

In some specific embodiments, the present invention provides the pharmaceutical composition in a solid form. The nanoparticle composition for rapid suspension comprises the active ingredient (e.g. paclitaxel) in a content of 1-30%, preferably 2-20% or 3-15% or 5-10% by weight. Such solid form pharmaceutical composition may be resuspended in a pharmaceutically acceptable carrier. The pharmaceutically acceptable carrier includes, but not limited to, a buffer, a preservative, water for injection, physiological saline, and isotonic solution. In some specific embodiments, this solid is resuspended in water for injection, preferably the resulting resuspension comprises the active ingredient (e.g., paclitaxel) in a content of about 5 mg/ml.

In a third aspect, the present invention further provides a method for preparing the above nanoparticle composition for rapid suspension, comprising the steps of: (1) dissolving the active ingredient in an organic solvent to form an oil phase, and dissolving the protein in water to form an aqueous phase, (2) mixing and homogenizing the oil phase and the aqueous phase to form a nanoemulsion, (3) removing the organic solvent in the nanoemulsion to obtain a suspension, (4) dialyzing, and optionally (5) lyophilizing.

In some embodiments, in step (1), the organic solvent is selected from the group consisting of pure solvents with low water solubility and low boiling point or a mixed solvent thereof with a small molecular alcohol. Preferably, the organic solvent is one or more selected from the group consisting of chloroform, dichloromethane, ethanol or tert-butyl alcohol, preferably chloroform or a combination of chloroform and ethanol. A skilled person can select an appropriate organic solvent based on the nature of the active ingredient. In some specific embodiments, when the active ingredient is a taxane, the organic solvent that can be used is one or more selected from the group consisting of chloroform and ethanol. More specifically, when the active ingredient is paclitaxel or docetaxel, the organic solvent that can be used is a mixed system of chloroform and ethanol. In some specific embodiments, the volume ratio of chloroform to ethanol is 1:1 to 20:1, preferably 1:1 to 15:1, preferably 1:1 to 11:1; for example, selected from the group consisting of 1:1, 4:1, 9:1 and 11:1.

In some specific embodiments, the concentration of the active ingredient in the oil phase ranges from 20 to 500 mg/ml, preferably from 50 to 350 mg/ml, preferably from 60 to 250 mg/ml, and preferably from 80 to 200 mg/ml. In some specific embodiments, the concentration of the active ingredient in the oil phase is selected from the group consisting of 20 mg/ml, 30 mg/ml, 50 mg/ml, 83 mg/ml, 100 mg/ml, 133 mg/ml, 150 mg/ml, 167 mg/ml, 180 mg/ml, 200 mg/ml, 250 mg/ml, 300 mg/ml, 333 mg/ml, 450 mg/ml and 500 mg/ml.

In some specific embodiments, the concentration of the protein in the aqueous phase is 2% to 10% (w/v), preferably 2% to 7% (w/v), preferably 2% to 5% (w/v), for example, selected from the group consisting of 2%, 4%, 5% and 10%.

In some embodiments, in the step (2), the oil phase and the water phase are mixed to form a nanoemulsion, and the volume ratio of the oil phase to the water phase is 1:10 to 1:100, preferably 1:10 to 1:60, more preferably 1:20 to 1:40. In some specific embodiments, the mixing ratio of the oil phase to the water phase is 3:100 or 1:25. The nanoemulsion can be formed using a method known in the art, including but not limited to homogenization. For example, the mixture of the oil phase and the water phase is homogenated by a high-shear disperser, and then homogenized by a high-pressure homogenizer to obtain an oil-in-water emulsion. In a specific embodiment, the mixture of the oil phase and the water phase is homogenated by a high shear disperser for 2-10 minutes, and then homogenized by a high-pressure homogenizer at 10,000-20,000 psi to obtain an oil-in-water emulsion.

In some embodiments, in step (3), the organic solvent in the nanoemulsion is removed, and any known suitable method may be used, such as evaporation under reduced pressure, dialysis, etc. Among them, evaporation under reduced pressure includes falling-film evaporation.

In some specific embodiments, falling-film evaporation is used to remove the organic solvent in the nanoemulsion. In a specific embodiment, the emulsion is evaporated by a falling-film evaporator at 60°C and 40 mbar to remove the organic solvent in the emulsion. After removing the organic solvent, the resulting suspension comprises the nanoparticles of the present disclosure. However, the suspension at this moment also comprised an excess amount of albumin, which did not actually participate in the formation of the nanoparticles.

In some specific embodiments, dialysis is used to remove the organic solvent in the nanoemulsion. In this case, step (3) can be combined with step (4).

In some embodiments, the dialyzing in the step (4) is performed in a dialysis fold of 2-10, preferably 3-6.

In some specific embodiments, the dialyzing is performed by adding a particle stabilizer to the suspension obtained in step (3), so that the concentration of the particle stabilizer in the suspension is 0.01%-1.4% (w/v), and dialyzing in an aqueous solution of a lyoprotectant. Preferably, the concentration of the particle stabilizer in the suspension is 0.1%-1.4% (w/v), preferably 0.1%-0.9% (w/v). Preferably, the aqueous solution of the lyoprotectant comprises 1%-10%, preferably 2%-8%, preferably 5%-6% of the lyoprotectant.

In some specific embodiments, the dialyzing is performed by dialyzing in an aqueous solution of a lyoprotectant, and the aqueous solution further comprises a particle stabilizer in a concentration of 0.01%-1.4% (w/v), preferably 0.05%-0.9% (w/v), preferably 0.05%-0.5% (w/v), more preferably 0.05%-0.15%. Preferably, the aqueous solution of the lyoprotectant comprises 1%-10%, preferably 2%-8%, preferably 5%-6% of the lyoprotectant.

In some specific embodiments, the dialyzing is performed by dialyzing in a solution of a particle stabilizer with an appropriate concentration of 0.01%-1.4% (w/v), preferably 0.1%-1.4% (w/v), preferably 0.1%-0.9% (w/v). Optionally, a lyoprotectant is added after dialysis, and preferably, the lyoprotectant is added until the resulting liquid comprises 1%-10%, preferably 2%-8%, more preferably 5%-6 % of the lyoprotectant.

In some specific embodiments, the lyophilizing in step (4) is performed by freezing between -20°C and -60°C and drying at a temperature between 0°C and +40°C.

The nanoparticle for rapid suspension involved in the present invention are intended to be administered via intravenous infusion, so the sterility of the product needs to be ensured. Both the nanoparticles and human serum albumin are temperature sensitive, so they cannot be sterilized by heat sterilization, and possible sterilization solutions include full aseptic production or sterilization by filtration.

In a fourth aspect, the present invention further provides a method of using the above nanoparticle for rapid suspension or the pharmaceutical composition. Because the nanoparticle for rapid suspension or the pharmaceutical composition can effectively transport multiple active ingredients, they can be used to treat diseases that are responsive to the active ingredient. For example, the nanoparticle for rapid suspension or the composition thereof can be used to treat cancer, such as liver cancer, prostate cancer, lung cancer, breast cancer, multiple myeloma, transplant rejection, colon cancer, and lymphoma. Other diseases further include fever and so on.

In a fifth aspect, the present disclosure further provides use of the above nanoparticle for rapid suspension or the pharmaceutical composition in the preparation of a medicament for treating a disease responsive to the active ingredient. For example, the disease includes cancer such as liver cancer, prostate cancer, lung cancer, breast cancer, multiple myeloma, transplant rejection, colon cancer, and lymphoma. Other diseases further include fever and so on.

Unless defined otherwise, all technical and scientific terms used herein have the same meaning as commonly understood by a commonly skilled person in the art.

Although the numerical ranges and parameter approximations shown in the present invention are within a wide range, the numerical values shown in the specific examples are recorded as accurately as possible. Any numerical value, however, inherently comprises certain errors resulting from the standard deviation in their respective measurements. Additionally, all ranges disclosed herein are to be understood to encompass any and all subranges contained therein. For example, a range recorded as "1 to 10" shall be deemed to include any and all subranges between the minimum value of 1 and the maximum value of 10, including the endpoints; that is, all subranges beginning with a minimum value of 1 or more, such as 1 to 6.1, and a subrange ending with a maximum value of 10 or less, such as 5.5 to 10. Additionally, any reference referred to as "incorporated herein" shall be understood to be incorporated in its entirety.

It should also be noted that, as used in this specification, the singular form includes the plural form of its referent, unless expressly and unambiguously limited to one referent. The term "or" is used interchangeably with the term "and/or" unless the context clearly dictates otherwise.

A skilled person understands that the particle size of particles may be measured by any existing or future suitable method, including but not limited to sedimentation, sieving, microscopy, or laser particle size analyzer. It should also be understood that, when the nanoparticles in the present disclosure are multiple, the particle size of each nanoparticle is not consistent, and it is also included in the scope of the present disclosure as long as its average particle size meets the above limitations. In some specific embodiments, the particle size is determined by a laser particle size analyzer.

The nanoparticle for rapid suspension or the pharmaceutical compositions of the present invention have one or more of the following advantages:
(1) Rapid suspension and less foaming.

The preparation before using the marketed albumin-bound paclitaxel Abraxane^{®} includes six steps and is complicated in operation, requiring a minimum of 8 minutes. The product dispersion time is long and the risk of contamination increases during drug preparation. The preparation operation takes up a lot of space and time, affecting the work efficiency of the preparation personnel; and when the preparation operation is irregular, it is easy to generate a lot of foam and occur incomplete dissolution, which may lead to problems such as inaccurate liquid metering and clogging of infusion lines, and may affect medication safety or lead to medical disputes.

The lyophilized product of the present invention can be quickly resuspended without complicated operations, shortening the reconstitution time of the preparation to be within 2 minutes, or even to 1 minute, and can reduce foaming and avoid problems such as incomplete dissolution, which greatly improves the convenience in clinical application and work efficiency and is beneficial to ensuring the safety of medication.

### (2) Good stability.

The nanoparticle for rapid suspension or the pharmaceutical composition of the present invention have good stability whether during the preparation process or after reconstitution and resuspension. When it is left at room temperature for 24 hours or even 48 hours, there are no obvious changes in liquid appearance, particle size, transmittance, etc. When the nanoparticle for rapid suspension or the pharmaceutical composition is placed under accelerated conditions for 10 days, there are no significant changes in particle size, transmittance, reconstitution time, etc.

### (3) Improved safety

Compared with the prior art, the present invention greatly reduces the amount of human serum albumin by optimizing the prescription formulation, not only maintains the functional structure and stability of the nanoparticles, but also can significantly reduce the allergic reaction caused by HSA. Compared with Abraxane^{®}, the product of the present invention has no significant difference in therapeutic effect while having significantly improved safety.

(4) The preparation method of the present invention is convenience and operable and suitable for large-scale production.

### BRIEF DESCRIPTION OF DRAWINGS

Fig. 1 shows average concentration-time curves of total paclitaxel in plasma after Beagle dogs were given lyophilized powder 4 and Abraxane (Mean ± SD, N=24).
Fig. 2 shows average concentration-time curves of free paclitaxel in plasma after Beagle dogs were given lyophilized powder 4 and Abraxane (Mean ± SD, N=24).
Fig. 3 shows tissue distribution results of human breast cancer JIMT-1 tumor-bearing mice after tail vein injection with 20 mg/kg lyophilized powder 4 and Abraxane^{®}.
Fig. 4 shows the inhibitory effect of drugs on human breast cancer JIMT-1 xenografts (*p<0.05, ***p<0.001, compared with the vehicle control group).
Fig. 5 shows the inhibitory effect of drugs on human breast cancer KPL-4 xenografts (***p<0.001, compared with the vehicle control group).
Fig. 6 shows the inhibitory effect of drugs on human pancreatic cancer CFPAC-1 xenografts (***p<0.001, compared with the vehicle control group).

### Detailed description of the invention

### Detection methods (I) methods for determining the contents of respective components of the product

### 1. Determination of the content of human serum albumin

The albumin content was determined by HPLC method at a detection wavelength of 228nm on a UV detector equipped with a Tosohaas TSK G3000 SWXL gel chromatographic column using a 0.1mol/L dipotassium hydrogen phosphate solution (adjusting pH to 7.0±0.1 by hydrochloric acid) as the mobile phase at a column temperature of 25 °C, a flow rate of 0.7ml/min and an injection volume of 10µl, and calculated using an external standard method.

Preparation of the test solution: The solution to be tested was diluted with a 0.9% sodium chloride solution to give a solution with the albumin concentration of less than 3 mg/ml, which was used as the test solution.

### 2. Determination of the content of paclitaxel

The paclitaxel content was determined by HPLC method on a UV detector equipped with an octadecylsilane bonded silica gel chromatographic column at a detection wavelength of 228nm using acetonitrile-water (1:1, v/v) as the mobile phase at a column temperature of 25°C, a flow rate of 1.0ml/min and an injection volume of 10 µl, and calculated using an external standard method.

Preparation of the test solution: The solution to be tested was diluted with acetonitrile until the paclitaxel is completely dissolved to obtain a solution with a concentration of 20-200 µg/ml, which was used as the test solution.

### 3. Determination of the content of mannitol

The mannitol content was determined by HPLC method on a differential refractive index detector equipped with a Sepax Carbomix Ca-NP chromatographic column using water as the mobile phase at a flow rate of 0.5 ml/min, a column temperature of 50°C, a detection temperature of the differential refractive index detector of 55°C and an injection volume of 20 µl, and calculated using an external standard method.

### 4. Determination of the content of sucrose

The sucrose content was determined by HPLC method on an evaporative light scattering detector equipped with an Agilent ZORBAX NH2 chromatographic column using acetonitrile-water (80:20, v/v) as the mobile phase at a flow rate of 1.5ml/min, a column temperature of 35°C, a nebulizer temperature of 55°C, a drift tube temperature of 55°C and a gas flow rate of 1.0ml/min for the evaporative light scattering detector, and an injection volume of 5µL, and calculated from the standard curve.

### 5. Determination of Sodium ion

The sodium ion content was determined on a conductivity detector equipped with a cation exchange chromatographic column (DionexIonPacTM CS16) using 30mM aqueous solution of methylsulfonic acid as the mobile phase at a flow rate of 0.4ml/min, a column temperature of 30°C and an injection volume of 25µL, and calculated using an external standard method.

### Detection method (II) Method for measuring transmittance

The change in transmittance of a transmittable product (transmitted light intensity ≥ 0.2%) can reflect the change in stability of the product. The smaller the change in transmittance within a certain period of time, the better the stability is. If the transmittance decreases, it indicates that the particles are aggregated; and if the transmittance increases, it indicates that the particles are settled.

The measurement was performed on a multiple light scattering instrument (Turbiscan, Formulaction), which scans the product at different times and monitors changes in the product's transmittance or backscattered light to determine whether the product has unstable such as aggregation, sedimentation, and stratification.

Preparation of sample cell: The sample to be tested was shaken evenly and put into a sample cell with a sample volume of about 20ml, and the sample cell was placed on a sample holder, and covered with a cap.

Sample measurement: The sample was measured by intelligent scanning by setting the scanning time to 24 hours and automatically performing a measurement every 30 seconds, and the transmittance values were selected at a fixed position and at different times.

The following examples are intended to better illustrate the compositions disclosed in the present invention, but are not intended to limit the present disclosure in any aspect.

It should be noted that the reagents and instruments used in the examples are from the same model and/or manufacturer.

### Example 1 Screening of the concentration of the particle stabilizer during preparation

20g of paclitaxel (CAS: 33069-62-4, Hainan Yew Pharmaceutical Co., Ltd.) was dissolved in 120ml of chloroform/ethanol (11:1, v/v) to obtain an oil phase, and 800ml of human serum albumin solution (CAS: 70024-90-7, Hebei Daan Pharmaceutical Co., Ltd.) (20% w/v) was added with water to 4L to obtain an aqueous phase. The oil phase and the aqueous phase were emulsified online by a high shear disperser (model F22E, FLUKO Equipment (Shanghai) Co., Ltd.) to form a primary emulsion, and then homogenized by a high-pressure homogenizer (model M-110EH30K, Microfluidics Company) at 10,000-20,000 psi to obtain a nanoemulsion. The nanoemulsion was transferred to a falling film evaporator (model APLS -70L, Shanghai Tofflon Science and Technology Co., Ltd.) at a heating temperature of 50°C and a vacuum degree of lower than 80mbar, and evaporated under reduced pressure to remove the organic solvent in the solution to obtain a translucent suspension having paclitaxel albumin nanoparticles with an average diameter of 114nm.

The obtained suspension was dialyzed by a 300kD ultrafiltration membrane. After 6-fold dialysis with the dialysis substances in the following table respectively, most of the free human serum albumin in the suspension was replaced to give purified nanoparticle suspensions 1-1 to 1-8.

The purified nanoparticle suspensions were subjected to transmittance measurement according to the above "Detection Method (II) Method for measuring transmittance", and left at room temperature for 24 hours to observe the appearances thereof. The results are as follows.

**Table 1 Results for Stability**

| Sample Name | | Purified nanopart icle suspensi on 1-1 | Purified nanoparti cle suspensio n 1-2 | Purified nanoparti cle suspensio n 1-3 | Purified nanoparti cle suspensio n 1-4 | Purified nanoparti cle suspensio n 1-5 | Purified nanoparti cle suspensio n 1-6 | Purified nanoparti cle suspensio n 1-7 | Purified nanopart icle suspensi on 1-8 |
|---|---|---|---|---|---|---|---|---|---|
| Substances for dialysis | | Water | 0.1% NaCl | 0.9% NaCl | 1.4% NaCl | 2.0% NaCl | 5.0% NaCl | 8.0% NaCl | 10% NaCl |
| Parti cle size (nm) | 0h | 114 | 110 | 111 | 111 | 110 | 111 | 112 | 113 |
| | 24h | -- | 110 | 110 | 110 | -- | -- | -- | -- |
| Appe aranc e | 0h | Milky white suspensio n | Milky white suspension | Milky white suspension | Milky white suspension | Milky white suspension | Milky white suspension | Milky white suspension | Milky white suspensio n |
| | 4h | Obvious precipitate s | Milky white suspension | Milky white suspension | Milky white suspension | Milky white suspension | Obvious precipitate s | Obvious precipitate s | Obvious precipitate s |
| | 24h | -- | Milky white suspension | Milky white suspension | Milky white suspension | Obvious precipitates | -- | -- | -- |
| Tran smitt ance | 0h | -- | 10.59 | 18.97 | 11.73 | -- | -- | -- | -- |
| | 24h | -- | 11.16 | 18.85 | 12.44 | -- | -- | -- | -- |

The results showed that there was no precipitation occurred after dialysis with 0.1-1.4% sodium chloride solutions and being left for 24 hours during the preparation, and the transmittance of the products did not change significantly compared with the paclitaxel albumin nanoparticles before dialysis, indicating that sodium chloride in a certain concentration range can stabilize the product, especially the 0.9% NaCl solution, which is the best, and NaCl with too high concentration is not conducive to particle stability.

### Example 2 Preparation and stability investigation of nanoparticle for rapid suspension comprising sodium chloride (different dialysis folds)

20g of paclitaxel was dissolved in 200ml of chloroform/ethanol (11:1, v/v) to obtain an oil phase, and 800g of human serum albumin solution (20% w/v) was added with water to 4L to obtain an aqueous phase. The oil phase and the aqueous phase were emulsified online by a high-shear disperser to form a primary emulsion, and then homogenized by a high-pressure homogenizer at 10,000-20,000 psi to obtain a nanoemulsion. The nanoemulsion was transferred to a falling film evaporator at a heating temperature of 50°C and a vacuum degree of lower than 80mbar, and evaporated under reduced pressure to remove the organic solvent in the solution to obtain a translucent suspension having paclitaxel albumin nanoparticles with an average diameter of 112nm.

The obtained suspension was dialyzed 4-folds in volume by a 300kD ultrafiltration membrane using 5% mannitol solutions (comprising 0, 0.05%, 0.1%, 0.2%, 0.3%, 0.4%, and 0.5% sodium chloride respectively) as the dialysis solutions. After dialysis, most of the free human serum albumin in the suspension was replaced to give purified nanoparticle suspensions 2-0 to 2-6. 20 ml of each of the purified nanoparticle suspensions was put into a vial for lyophilizing to obtain nanoparticle lyophilized powder for rapid suspension 2-0 to 2-6 as white cakes.

The accelerated stability of the lyophilized powders was investigated, and the results are shown in Table 2.

The nanoparticle lyophilized powder for rapid suspension 2-0 to 2-6 obtained above were added with 20ml of water for injection respectively. The vials were swirled and/or inverted gently to allow all the lyophilized cakes/powders in the vials to be completely dispersed and dissolved, and the white cakes was dissolved completely within about 1 minute. Compared with the nanoparticle suspensions before lyophilization (having a particle size of 112nm), the particle size of the suspensions obtained by resuspending the lyophilized powders 2-1 to 2-6 did not change significantly, while the particle size of the sample 2-0 after lyophilization and reconstitution increased to 137 nm. The reconstituted suspensions of the above samples were left at room temperature for 24 hours, and no significant changes were observed in terms of the appearance and transmittance of the solutions, indicating that the addition of sodium chloride during dialysis is helpful to maintain the stability of the particle size during the process of lyophilization and reconstitution.

The lyophilized powders 2-0 to 2-6 were left at a high temperature of 60°C for 5 days, and the white cakes were completely dissolved within 2 minutes. The reconstituted samples were left at room temperature for 24 hours without obvious change in the appearance of all samples; and for 48 hours without precipitation in samples 2-1 and 2-2.

**Table 2 results of accelerated stability of nanoparticle lyophilized powder for rapid suspension**

| Sample Name | | lyophilize d powder 2-0 | lyophiliz ed powder 2-1 | lyophiliz ed powder 2-2 | lyophiliz ed powder 2-3 | lyophiliz ed powder 2-4 | lyophiliz ed powder 2-5 | lyophiliz ed powder 2-6 |
|---|---|---|---|---|---|---|---|---|
| NaCl (%) in the final product | | 0 | 0.8 | 1.6 | 3.1 | 4.6 | 6.0 | 7.4 |
| lyophil ized powde r (0 day) | Reconstitution time (s) | 67 | 66 | 54 | 47 | 51 | 44 | 48 |
| | Particle size (nm) | 137 | 125 | 116 | 117 | 117 | 119 | 121 |
| | Appearance (48h) | Milky white suspensio n | Milky white suspensi on | Milky white suspensi on | Milky white suspensi on | Milky white suspensi on | Milky white suspensi on | Milky white suspensi on |
| lyophil ized powde r (60°C , 5 days) | Reconstitution time (s) | 61 | 58 | 40 | 40 | 31 | 57 | 32 |
| | Particle size (nm) | 143 | 126 | 136 | 147 | 169 | 160 | 184 |
| | Appearance at 48h | Precipitat es observed | No precipita tes | No precipita tes | Obvious precipita tes | Obvious precipita tes | Obvious precipita tes | Obvious precipita tes |

### Example 3 Screening of different lyoprotectants

20g of paclitaxel was dissolved in 200ml of chloroform/ethanol (11:1, v/v) to obtain an oil phase, and 800g of human serum albumin solution (20% w/v) was added with water to 4L to obtain an aqueous phase. The oil phase and the aqueous phase were emulsified online by a high-shear disperser to form a primary emulsion, and then homogenized by a high-pressure homogenizer at 10,000-20,000 psi to obtain a nanoemulsion. The nanoemulsion was transferred to a falling film evaporator at a heating temperature of 50°C and a vacuum degree of lower than 80mbar, and evaporated under reduced pressure to remove the organic solvent in the solution to obtain a translucent suspension having paclitaxel albumin nanoparticles with an average diameter of 122nm.

An appropriate amount of sodium chloride was added to the obtained suspension such that the concentration of NaCl was 0.9%. Dialysis was performed 3-folds in volume by a 300kD ultrafiltration membrane with 5 dialysis solutions comprising different lyoprotectants in the table below to obtain purified nanoparticle suspensions 3-1 to 3-5. The results showed that the particle size did not change significantly during dialysis.

The purified nanoparticle suspensions 3-1 to 3-5 were packaged and lyophilized in a lyophilizer for 40 hours to obtain stable white cakes, which are the nanoparticle lyophilized powder for rapid suspension 3-1 to 3-5.

The nanoparticle lyophilized powder for rapid suspension 3-1 to 3-5 were added into water and resuspended to become isotonic solutions. The white cakes could be completely dissolved within 2 minutes. There is no significant change when the resuspended lyophilized powders were left at room temperature for 24 hours, and the resuspended suspension of the sample obtained by dialyzing with a 5% mannitol + 1% sucrose solution and lyophilizing was more stable when left at room temperature, and no significant change was observed after being left for 48 hours.

**Table 3 Screening of Lyoprotectant types and combination**

| Type of dialysis solution | Suspension | | Resuspended lyophilized powder | |
|---|---|---|---|---|
| | Name | Particle size/nm | Name | Particle size/nm |
| 5% mannitol | Purified nanoparticle suspension 3-1 | 122 | Nanoparticle lyophilized powder for rapid suspension 3-1 | 124 |
| 1% mannitol | Purified nanoparticle suspension 3-2 | 122 | Nanoparticle lyophilized powder for rapid suspension 3-2 | 123 |
| 5% mannitol + 1% sucrose | Purified nanoparticle suspension 3-3 | 121 | nanoparticle lyophilized powder for rapid suspension 3-3 | 120 |
| 1% mannitol + 1% sucrose | Purified nanoparticle suspension 3-4 | 122 | nanoparticle lyophilized powder for rapid suspension 3-4 | 123 |
| 4% mannitol + 2% sucrose | Purified nanoparticle suspension 3-5 | 122 | nanoparticle lyophilized powder for rapid suspension 3-5 | 124 |

The nanoparticle lyophilized powder for rapid suspension 3-1 to 3-5 were left at a high temperature of 60°C for 10 days, and transmittance was measured according to the "Detection Method (II) Method for measuring transmittance" and the average particle size was measured. The results are shown in the table below.

**Table 4 results of accelerated stability of purified nanoparticle suspension and lyophilized powders**

| Type of dialysis solution | | lyophilized powder 3-1 | lyophilized powder 3-2 | lyophilized powder 3-3 | lyophilized powder 3-4 | lyophilized powder 3-5 |
|---|---|---|---|---|---|---|
| Drug solution after dialysis | Drug concentration(m g/ml) | 5.91 | 5.77 | 6.15 | 6.23 | 6.05 |
| | Protein concentration(m g/ml) | 11.82 | 10.90 | 14.15 | 12.46 | 12.28 |
| | Protein: Drug | 2.10 | 1.89 | 2.30 | 2.00 | 2.03 |
| | Particle protective agent | 0.04 | 0.04 | 0.04 | 0.04 | 0.04 |
| | Particle size (nm) | 122 | 122 | 122 | 122 | 122 |
| | Transmittance% (0h) | 11.29 | 13.68 | 13.45 | 13.78 | 11.99 |
| | Transmittance% (24h) | 11.43 | 13.29 | 13.46 | 13.81 | 11.90 |
| lyophilize d powder (0 day) | Cake Appearance | White cake with a lot of crystals | White cake with a small volume and a lot of crystals | White cake | White cake | White cake |
| | Reconstitution time (s) | 77 | 65 | 67 | 90 | 45 |
| | Particle size (nm) | 124 | 123 | 120 | 124 | 123 |
| | Transmittance% (0h) | 15.83 | 13.68 | 18.14 | 15.59 | 15.50 |
| | Transmittance% 24h) | 15.94 | 13.29 | 18.42 | 15.39 | 15.32 |
| lyophilize d powder (accelerat ed at 60°C, 5 days) | Reconstitution time (s) | 56 | 64 | 49 | 77 | 63 |
| | Particle size (nm) | **129** | **129** | 123 | **126** | 124 |
| | Transmittance% (0h) | 13.97 | 12.37 | 18.44 | 14.21 | 14.23 |
| | Transmittance% (24h) | 14.78 | 13.28 | 18.76 | 14.46 | 14.32 |
| lyophilize d powder (accelerat ed at 60°C, 10 days) | Reconstitution time (s) | 52 | 62 | 64 | 69 | 37 |
| | Particle size (nm) | **132** | **132** | 121 | **128** | 125 |
| | Transmittance% (0h) | 13.33 | 12.38 | 17.19 | 13.69 | 14.10 |
| | Transmittance% (24h) | 14.09 | 13.58 | 17.23 | 14.26 | 14.23 |

The results showed that mannitol as a lyoprotectant can protect purified paclitaxel albumin nanoparticles, and the addition of sucrose can better ensure the stability of paclitaxel nanoparticles during lyophilization (little change in transmittance between 0h and 24h). The combination of mannitol/sucrose as the lyoprotectant has a good protective effect on both the feed solutions during dialysis and the lyophilized products.

After left under accelerated conditions, lyophilized powders 3-1, 3-2, 3-4, and 3-5 after reconstitution showed particle size increase in different degrees, while the particle size and transmittance of lyophilized powder 3-3 remained unchanged, indicating that 5% mannitol + 1% sucrose as a lyoprotectant had the best effect and no additional osmotic osmotic pressure adjuster was required to meet the requirements.

In addition, similar to lyophilized powder 3-3, the particle size and transmittance of lyophilized powders 3-4 and 3-5 remain basically unchanged, and 1% mannitol + 1% sucrose and 4% mannitol + 2% sucrose as lyoprotectants were also the best.

### Example 4 Preparation of nanoparticle lyophilized powder for rapid suspension

50g of paclitaxel was dissolved in 500ml of chloroform/ethanol (11:1, v/v) to obtain an oil phase, and 2.08kg of human serum albumin solution (20% w/v) was added with water to 10L to obtain an aqueous phase. The oil phase and the aqueous phase were emulsified online by a high-shear disperser to form a primary emulsion, and then homogenized by a high-pressure homogenizer at 10,000-20,000 psi to obtain a nanoemulsion. The nanoemulsion was transferred to a falling film evaporator at a heating temperature of 50°C and a vacuum degree of lower than 80mbar, and evaporated under reduced pressure to remove the organic solvent in the solution to obtain a translucent suspension having paclitaxel albumin nanoparticles with an average diameter of 118nm.

An appropriate amount of sodium chloride was added to the obtained suspension such that the concentration of NaCl was 0.9%. Dialysis was performed 4-folds in volume by a 300kD ultrafiltration membrane with 5% mannitol + 1% sucrose. The dialyzed paclitaxel albumin nanoparticles had an average diameter of 121 nm, and purified nanoparticles 4 as a white translucent liquid was obtained.

The purified nanoparticle suspension after dialysis was filtered smoothly through a 0.22 micron sterilization filter (Sartorius, Germany). The particle size did not change significantly after filtration, and no significant change was observed when the suspension was left at room temperature for 48 hours. The suspension was packaged and then lyophilized in a lyophilizer for 80 hours to obtain a stable white cake, which is the nanoparticle lyophilized powder for rapid suspension 4 (referred to as lyophilized powder 4).

The white cake obtained by lyophilization was added with water for injection to become an isotonic solution, and shaken gently to be dissolved completely. The white cake could be completely dissolved in about 1 minute with slight foaming. The resuspended suspension was left at room temperature for 48 hours without significant changes.

### Example 5 Preparation of more batches of nanoparticle lyophilized powder for rapid suspension

The method in Example 4 was used to prepare three batches of nanoparticle lyophilized powder for rapid suspension, named 5-1, 5-2 and 5-3 respectively. The sample names and main formulations and processes are shown in the table below.

**Table 5 Preparation of nanoparticle lyophilized powder for rapid suspension 17, 18 and 19**

| Name | Dialysis solution | Process | Particle size (nm) | Name after lyophilization |
|---|---|---|---|---|
| Purified nanoparticle suspension 5-1 | 5% mannitol + 1% sucrose | 3-fold dialysis | 118 | nanoparticle lyophilized powder for rapid suspension 5-1 |
| Purified nanoparticle suspension 5-2 | | | 120 | nanoparticle lyophilized powder for rapid suspension 5-2 |
| Purified nanoparticle suspension 5-3 | | | 122 | nanoparticle lyophilized powder for rapid suspension 5-3 |

### Example 6 Screening of Protein to Drug Ratio

The purified nanoparticle suspension 3-3 obtained in Example 3 was dialyzed at different folds. After each dialysis, the contents of human serum albumin and paclitaxel were determined according to "Detection methods (I) methods for determining the contents of respective components of the product" to obtain protein-drug ratio. The products after each dialysis were lyophilized, and then added with 20ml of water for injection, and shaken gently to be dissolved completely. The reconstitution dispersion time and foaming of the lyophilized samples at different dialysis folds were observed. The results are as follows.

**Table 6 Dispersion time and foaming of lyophilized samples at different dialysis folds**

| | Before dialysis | 1-fold dialysis | 2-fold dialysis | 3-fold dialysis | 4-fold dialysis | 5-fold dialysis | 6-fold dialysis |
|---|---|---|---|---|---|---|---|
| Protein-drug ratio | 8.95 | 5.63 | 3.69 | 2.30 | 1.49 | 1.03 | 0.70 |
| Foaming | +++ | +++ | ± | ± | ± | ± | ± |
| Dispersion time (s) | 439 | 355 | 127 | 62 | 46 | 43 | 57 |

| | | | | | | | |
|---|---|---|---|---|---|---|---|
| Note: The foaming is represented by +, more + means more foam. ± means there is slight foam. - indicates no foam. | | | | | | | |

The results show that dialysis can effectively reduce the protein-to-drug ratio in purified nanoparticle suspension. As the dialysis fold increases, the reconstitution time of the product is significantly shortened, and the foaming is significantly improved. Combined with Example 3, when the albumin/paclitaxel ratio is less than 3, the dispersion time of the product is about 1 minute, and the foam generated is significantly reduced. Further increased dialysis fold can further reduce the protein-to-drug ratio, without obvious improvement in dispersion time and foaming.

### Example 7 Stability investigation under high temperature

The paclitaxel albumin nanoparticle suspension (before dialysis) prepared as described in the first paragraph of Example 4 was lyophilized and left together with the nanoparticle lyophilized powder for rapid suspension 4 under accelerated conditions of 40°C and 60°C for 5 days and 10 days. After taking out, they were reconstituted with 0.9% sodium chloride solution and 20ml of water for injection respectively. The changes in dispersion time, particle size and transmittance were recorded, and the results are as follows.

**Table 7 Stability of nanoparticle lyophilized powders for rapid suspension and paclitaxel albumin nanoparticles under accelerated conditions**

| Sample Name | Storage conditions | Reconstitution time | Particle size | Transmittance at 0h | Transmittance at 24h |
|---|---|---|---|---|---|
| lyophilized powder 4 | 0 day | 1 minute 5 seconds | 115 | 18.13 | 18.14 |
| | 40°C, 5 days | 51 seconds | 115 | 18.98 | 19.48 |
| | 40°C, 10 days | 1 minute 14 seconds | 117 | 19.76 | 20.31 |
| | 60°C, 5 days | 46 seconds | 116 | 18.95 | 19.53 |
| | 60°C,10 days | 58 seconds | 117 | 19.49 | 20.20 |
| Paclitaxel albumin nanoparticle lyophilized powder (before dialysis) | 0 day | 8 minutes 9 seconds | 115 | 11.74 | 11.94 |
| | 40°C, 5 days | 8 minutes 11 seconds | 116 | 11.92 | 12.49 |
| | 40°C, 10 days | 7 minutes 45 seconds | 116 | 12.43 | 13.88 |
| | 60°C, 5 days | 8 minutes 32 seconds | 117 | 10.21 | 10.68 |
| | 60°C, 10 days | 8 minutes 7 seconds | 116 | 11.17 | 12.09 |

It can be seen from the above results that after stored under accelerated conditions, the nanoparticle lyophilized powder for rapid suspension 4 has no significant differences in particle size after isotonic reconstitution and transmittance at 24h and no disadvantage in stability, with significantly accelerated reconstitution speed and significantly reduced foam, compared with the paclitaxel albumin nanoparticle lyophilized powder.

### Example 8 Comparison of reconstitution phenomenon

The nanoparticle lyophilized powder for rapid suspension4 in Example 4 was added with 20 ml of injection water, and shaken gently to completely dissolve. The white cake was completely dissolved in 24 seconds with only slight foaming.

Two vials of commercially available Abraxane were taken. One of them was dispersed by adding 20 ml of 0.9% sodium chloride solution according to the standard operation in the package insert. It took 10 minutes for the sample to be completely dissolved with slight foaming. The other one was added with 0.9% sodium chloride without controlling the injection angle and speed, and as a result, it was still not completely dispersed after 23 minutes, white solids were visible and more foam was generated.

The reconstituted suspension of the nanoparticle lyophilized powder 4 and Abraxane were left at room temperature to evaluate the stability, and both of them have no significant change within 72 hours.

The results show that Abraxane has a complicated liquid preparation operation before use, and improper operation can easily cause problems such as excessive foaming and incomplete dissolution. The particle dispersion in the present invention does not require complicated operating steps, and has a dispersion time of less than 1 minute, which is significantly shorter than Abraxane, and significantly reduced foaming.

### Example 9 Comparative Study on Quality

The nanoparticle lyophilized powder for rapid suspension 5-1 to 5-3 in Example 5 were compared with the commercially available Abraxane in quality, and the comparison items included appearance, pH, moisture, dispersion time, average particle size, total impurities, human serum albumin, human serum albumin polymer, paclitaxel binding rate, in vitro release rate and content.

**Table 8 Comparison of nanoparticle lyophilized powder for rapid suspension with paclitaxel albumin nanoparticles in quality**

| Testing items | nanoparticle lyophilized powder for rapid suspension 5-1 | nanoparticle lyophilized powder for rapid suspension 5-2 | nanoparticle lyophilized powder for rapid suspension 5-3 | Abraxane |
|---|---|---|---|---|
| Appearance | White lyophilized cake | White lyophilized cake | White lyophilized cake | White lyophilized cake |
| pH | 7.2 | 7 | 7.2 | 6.8 |
| Moisture (%) | 1 | 0.8 | 1 | 1.7 |
| Dispersion time (min) | 1 | 1 | 1 | 10 |
| Average particle size | 121 | 121 | 120 | 132 |
| Total impurities | 0.08 | 0.12 | 0.13 | 0.44 |
| Human serum albumin (mg/vial) | 245 | 233 | 239 | 870 |
| Human serum albumin polymer (mg/vial) | 21 | 21 | 21 | 27 |
| Paclitaxel binding rate (%) | 94 | 94 | 94 | 95 |
| In vitro release rate (%) | 96 | 96 | 97 | 93 |
| Content (mg/vial) | 100.9 | 99.2 | 100.4 | 104.8 |

The results show that the composition of the present invention has significantly reduced human serum albumin content and reconstitution time, and lowered total impurities and human serum albumin polymer content, which are better than Abraxane, and the other indicators are basically the same.

### Example 10 Comparative study on high temperature stability

The nanoparticle lyophilized powder for rapid suspension 5-1 in Example 5 was left at 60°C and compared with a commercial Abraxane. The comparison items included appearance, pH, moisture, dispersion time, average particle size, related substances, human serum albumin, human serum albumin polymer, paclitaxel binding rate, in vitro release rate and content.

**Table 9 High temperature stability of nanoparticle lyophilized powder for rapid suspension and paclitaxel albumin nanoparticles**

| Testing items | 60°C, 5 days | | 60°C, 10 days | |
|---|---|---|---|---|
| | nanoparticle lyophilized powder for rapid suspension 5-1 | Abraxane | nanoparticle lyophilized powder for rapid suspension 5-1 | Abraxane |
| Appearance | White lyophilized cake | Yellowish lyophilized cake | White lyophilized cake | Yellowish lyophilized cake |
| pH | 7.2 | 6.6 | 7.2 | 6.7 |
| Moisture(%) | 0.99 | 2 | 1 | 1.7 |
| Dispersion time (min) | 1 | 11 | 1 | 10 |
| Average particle size | 121 | 145 | 121 | 147 |
| related substances | 0.54 | 0.85 | 0.71 | 1.19 |
| Human serum albumin (mg/vial) | 241 | 815 | 242 | 790 |
| Human serum albumin polymer (mg/vial) | 18 | 33 | 19 | 32 |
| Paclitaxel binding rate (%) | 95 | 96 | 95 | 96 |
| In vitro release rate (%) | 94 | 95 | 96 | 95 |
| Assay (%) | 99.2 | 98.1 | 98.4 | 97 |

The results show that, after left under high temperature for 10 days, both of the nanoparticle lyophilized powder for rapid suspension of the present invention and Abraxane had increased total impurities, and the increasing degree was consistent; Abraxane had decreased human serum albumin, and the nanoparticle lyophilized powder for rapid suspension of the present invention had no significant change in protein content, indicating that the nanoparticle lyophilized powder for rapid suspensionof the present invention had more stable human serum albumin than Abraxane under high temperature.

### Example 11 Comparative study on light stability

The nanoparticle lyophilized powder for rapid suspension 5-1 in Example 5 was left under light and compared with a commercial Abraxane. The comparison items included appearance, pH, moisture, dispersion time, average particle size, related substances, human serum albumin, human serum albumin polymer, paclitaxel binding rate, in vitro release rate and content.

**Table 10 Light stability of nanoparticle lyophilized powder for rapid suspension and paclitaxel albumin nanoparticles**

| Testing items | Light exposure for 5 days | | Light exposure for 10 days | |
|---|---|---|---|---|
| | nanoparticle lyophilized powder for rapid suspension 5-1 | Abraxane | nanoparticle lyophilized powder for rapid suspension 5-1 | Abraxane |
| Appearance | White lyophilized cake | Yellowish lyophilized cake | White lyophilized cake | Yellowish lyophilized cake |
| pH | 7.2 | 6.6 | 7.2 | 6.7 |
| Moisture (%) | 1 | 1.7 | 0.92 | 1.9 |
| Dispersion time (min) | 1 | 10 | 1 | 10 |
| Particle size (nm) | 120 | 141 | 120 | 141 |
| Total impurities | 0.09 | 0.69 | 0.12 | 0.67 |
| Human serum albumin (mg/vial) | 242 | 867 | 239 | 858 |
| Human serum albumin polymer (mg/vial) | 19 | 35 | 19 | 34 |
| Paclitaxel binding rate (%) | 95 | 96 | 95 | 96 |
| In vitro release rate (%) | 94 | 94 | 94 | 94 |
| Content (%) | 99.3 | 100.2 | 98.4 | 97.2 |

The results show that, after being left under light for 10 days, the nanoparticle lyophilized powder for rapid suspension of the present invention has no significantly increased total impurities, which was significantly lower than Abraxane; and for the other indicators, there is no significant change trend for respective products.

### Example 12 Determination of the content of each component in the nanoparticle lyophilized powder for rapid suspension

The nanoparticle lyophilized powder for rapid suspension in Examples 4 to 5 were determined in terms of the contents of mannitol, sucrose and sodium ion according to "Detection methods (I) methods for determining the contents of respective components of the product".

**Table 11 Test results of respective components in the nanoparticle lyophilized powder for rapid suspension**

| Sample | Albumin (mg/vial) | Paclitaxel (mg/vial) | Mannitol (mg/vial) | Sucrose (mg/vial) | Sodium ion (mM) | Content of sodium chloride*(%) |
|---|---|---|---|---|---|---|
| Nanoparticle lyophilized powder for rapid suspension 2-1 | 153 | 100.5 | 933.7 | 0 | 170.9 | 0.8 |
| Nanoparticle lyophilized powder for rapid suspension 4 | 138 | 102.7 | 963.0 | 192.6 | 0.10 | 0.008 |
| Nanoparticle lyophilized powder for rapid suspension 5-1 | 245 | 100.9 | 923.9 | 189.2 | 0.28 | 0.022 |
| Nanoparticle lyophilized powder for rapid suspension 5-2 | 233 | 99.2 | 933.9 | 190.3 | 0.28 | 0.022 |
| Nanoparticle lyophilized powder for rapid suspension 5-3 | 239 | 100.4 | 925.9 | 190.7 | 0.28 | 0.022 |

| | | | | | | |
|---|---|---|---|---|---|---|
| Note: *calculated based on sodium ion content. | | | | | | |

### Example 13. Pharmacokinetics study in dogs

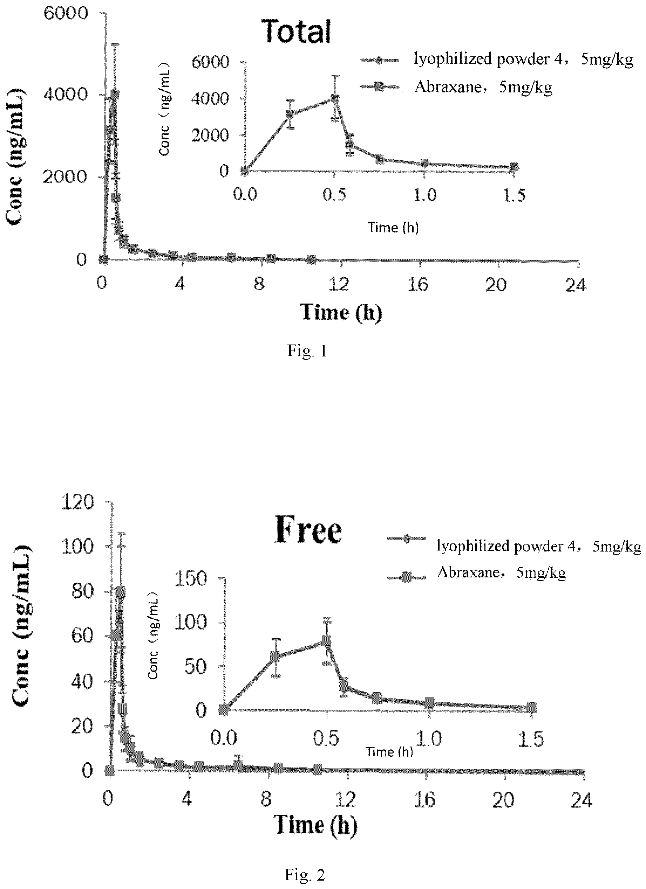

The test drugs were commercially available preparation Abraxane and lyophilized powder 4 in Example 4, and beagle dogs were used to conduct in vivo pharmacokinetic studies of the two preparations. The dosage is 5 mg/kg, and the administration time is controlled within 30 minutes. 2 ml of blood was collected from the cephalic vein of the beagle's forelimb before administration (0h), 15min during infusion (0.25h from the beginning of administration), at the needle withdrawal point (0.5h from the beginning of administration), and 0.58, 0.75, 1.0, 1.5, 2.5, 3.5, 4.5, 6.5, 8.5, and 24.5 hours from the beginning of administration, respectively, placed in heparin anticoagulant tubes, shaken evenly and centrifuged at 3000 rpm for 10 min to separate the plasma. HPLC/MS/MS was used to determine the concentration of paclitaxel in plasma to obtain the pharmacokinetic parameters, and drug concentration versus time curves were plotted as shown in Figs. 1 to 2. The main pharmacokinetic parameters are shown in Tables 12 to 13. It can be seen from the drug concentration-time curves of the two preparations that they have almost identical in vivo behaviors.

### Example 14. Allergy phenomena in pharmacokinetic studies in dogs

In the pharmacokinetics study in dogs conducted in Example 13, the adverse reactions of the two preparations to dogs were observed during administration. In the Abraxane group, all the tested beagle dogs (100%) had obvious allergic reactions to varying degrees, mainly including violent struggle during administration, excessive salivation, skin erythema around the mouth, and vomiting and urinary incontinence during the administration of some experimental animals. However, only some animals (50%) in the nanoparticle lyophilized powder for rapid suspension 4 group showed slight struggling, salivation, and skin erythema around the mouth, and the symptoms were mild. It can be seen that the product of the present invention can significantly reduce the allergic reaction of the drug.

### Example 15 Tissue distribution

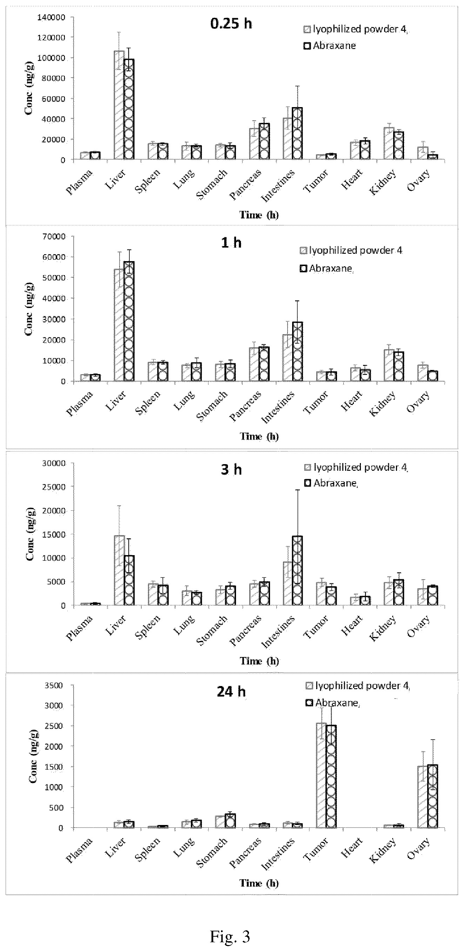

The test drugs were commercially available preparation Abraxane and the nanoparticle lyophilized powder for rapid suspension 4 in Example 4. 48 human breast cancer JIMT-1 tumor-bearing mice were randomly divided into 2 experimental groups, with a total of 6 mice in each experimental group at each time point. Both of the two preparations were administered to the respective experimental mice at a dose of 20 mg/kg through tail vein injection. Blood samples were collected from each group at 0.25, 1, 3 and 24 hours after administration and the mice were sacrificed immediately. Tumor, heart, liver, spleen, lung, kidney, stomach, small intestine, pancreas and ovary tissues were dissected and subjected to pretreatments such as homogenization, the drug concentration in each tissue sample was determined by protein precipitation-LC/MS/MS method, and the main pharmacokinetic parameters were calculated by using the non-compartmental model of Phoenix WinNonlin 8.0 software (Pharsight, USA). The tissue distribution results are shown in Fig. 3.

The results showed that, paclitaxel was widely distributed in mice, and could be detected in tumor, liver, lung, heart, stomach, small intestine, spleen and kidney tissues, with the highest exposure in the liver. According to the drug exposure in vivo, the two preparations had basically consistent exposure in various tissues of mice, without significant differences.

### Example 16 Inhibitory effect on human breast cancer JIMT-1 tumor

Experimental model: The NU/NU nude mouse model bearing human breast cancer JIMT-1 was used to investigate the inhibitory effect of purified particle lyophilized powder 4 on human breast cancer JIMT-1, and compared with the same dose of Abraxane.

Experimental method: 70 NU/NU female mice were subcutaneously inoculated with human breast cancer JIMT-1 cells (1×10⁷/0.1mL/mouse) in the axilla of the right forelimb. On Day 10 after inoculation, the average tumor volume grew to approximately 165 mm³. 40 Animals with good tumor growth were selected and divided equally into 5 groups (Day0) according to the tumor volume, with 8 animals in each group, including a vehicle control group, 10, 20 and 40mg/kg groups of lyophilized powder 4, and 20mg/kg group of Abraxane^{®}. The drug was administered intravenously once a week for a total of 3 times (abbreviated as qw×3, that is, administered on D0, D7, and D14), and the experiment ended on Day 24.

SPSS 19.0 statistical software was used for data processing, and the Repeated Measure process was used to analyze the changes in tumor volume between multiple measurements over time.

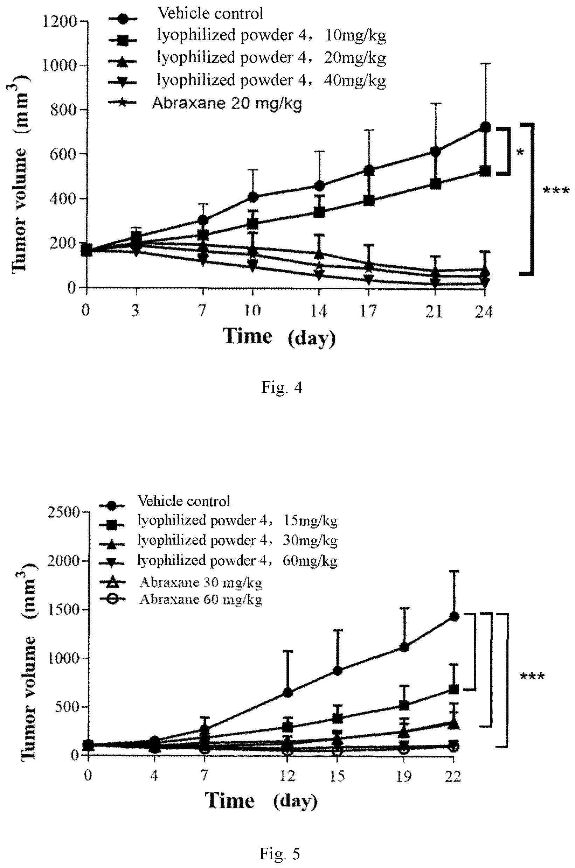

The results showed that, 1) the animals in each administration group tolerated well after intravenous administration in qw×3 to JIMT-1 tumor-bearing mice; 2) under the experimental conditions, intravenous administration of lyophilized powder 4 at 10, 20, and 40 mg/kg could significantly inhibit the growth of human breast cancer JIMT-1 xenograft in a dose-dependent manner, compared with the vehicle control group; 3) the inhibitory effect of lyophilized powder 4 on the tumor is equivalent to that of Abraxane at the same dose (20 mg/kg). The tumor growth curves of each group are shown in Fig. 4.

### Example 17 Inhibitory effect on human breast cancer KPL-4 tumor

Experimental model: The NU/NU nude mouse model bearing human breast cancer KPL-4 was used to investigate the inhibitory effect of purified particle lyophilized powder 4 on human breast cancer KPL-4, and compared with the same dose of Abraxane.

Experimental method: 70 NU/NU female mice were subcutaneously inoculated with human breast cancer KPL-4 cells (1×10⁷/0.1mL/mouse) in the axilla of the right forelimb. On Day 12 after inoculation, the average tumor volume grew to approximately 105mm³. 48 Animals with good tumor growth were selected and divided equally into 6 groups (Day0) according to the tumor volume, including a vehicle control group, 15, 30 and 60mg/kg groups of lyophilized powder 4, and 30 and 60mg/kg groups of Abraxane^{®}. The dosing frequency was qw×3, and the experiment ended on D22.

SPSS 19.0 statistical software was used for data processing, and the Repeated Measure process was used to analyze the changes in tumor volume between multiple measurements over time.

The results showed that, 1) under the experimental conditions, intravenous administration of lyophilized powder 4 at 15, 30, and 60 mg/kg could significantly inhibit the growth of human breast cancer KPL-4 xenograft in a dose-dependent manner, compared with the vehicle control group; 2) the inhibitory effect of lyophilized powder 4 on the tumor is equivalent to that of Abraxane at the same doses (30 mg/kg and 60 mg/kg). The tumor growth curves of each group are shown in Fig. 5.

### Example 18 Inhibitory effect on human pancreatic cancer CFPAC-1 tumor

Experimental model: The NU/NU nude mouse model bearing human pancreatic cancer CFPAC-1 was used to investigate the inhibitory effect of lyophilized powder 4 on human pancreatic cancer CFPAC-1, and compared with the same dose of Abraxane.

Experimental method: 70 NU/NU female mice were subcutaneously inoculated with human pancreatic cancer CFPAC-1 cells (1×10⁷/0.1mL/mouse) in the axilla of the right forelimb. On Day 7 after inoculation, the average tumor volume grew to approximately 195mm³. 35 Animals with good tumor growth were selected and divided equally into 5 groups (Day0) according to the tumor volume, including a vehicle control group, 10, 20, and 40 mg/kg groups of lyophilized powder 4, and 20mg/kg group of Abraxane^{®}. The dosing frequency was qw×3, and the experiment ended on D23.

SPSS 19.0 statistical software was used for data processing, and the Repeated Measure process was used to analyze the changes in tumor volume between multiple measurements over time.

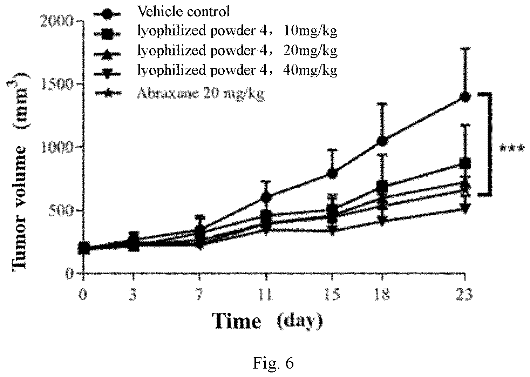

The results showed: 1) the animals in each administration group tolerated well after intravenous administration in qw×3 to CFPAC-1 tumor-bearing mice; 2) under the experimental conditions, intravenous administration of lyophilized powder 4 at 10, 20, and 40 mg/kg can significantly inhibit the growth of human pancreatic cancer CFPAC-1 xenograft in a dose-dependent manner, compared with the vehicle control group; 3) the inhibitory effect of lyophilized powder 4 on the tumor is equivalent to that of Abraxane at the same dose (20 mg/kg). The tumor growth curves of each group are shown in Fig. 6.

### Example 19 Toxicity test of repeated intravenous injection for three weeks in Beagle dogs

For this experiment, 42 Beagle dogs, half male and half female, were randomly divided into 7 groups, including a lyophilized powder 4 low-dose group (0.3mg/kg), a lyophilized powder 4 medium-dose group (0.6mg/kg), a lyophilized powder 4 high dose group (1.2mg/kg), an Abraxane^{®} group (1.2mg/kg), a negative control group (0.9% sodium chloride injection), an excipient control group-1 (human serum albumin 1.7mg/kg, consistent with the protein content of the lyophilized powder 4 high-dose group), and an excipient control group-2 (human serum albumin 10.8 mg/kg, consistent with the protein content of the Abraxane^{®} group). The drug was administered by intravenous injection at equal concentrations (5 mg/ml) and unequal volumes, once a week for a total of 3 times.

The results showed that, during the experiment, no animals died/was dying, and no significant abnormalities were found in body weight, food intake, body temperature, hematology and coagulation, serum biochemistry, urine, fecal occult blood, and gross anatomy.

In the excipient control group-2, 2/3 female animals vomited yellow foam after administration on D15, and in the commercial control group, 1/3 male animals had allergic reactions such as inability to stand and reduced activity (recovered after intramuscular injection of dexamethasone for emergency treatment). No allergic-related reactions were observed in the other dose groups.

At equal doses, there were no statistical difference in paclitaxel exposure (Cₘₐₓ and AUC₍₀₋ₜ₎) in female or male dogs after administration of lyophilized powder 4 and Abraxane^{®} on D1 and D15.

Conclusion: Under the experimental conditions, lyophilized powder 4 and Abraxane^{®} have similar in vivo PK characteristics, and lyophilized powder 4 has significantly reduced allergic reactions.

### Example 20. In vitro hemolysis experiment

In vitro hemolysis experiment was conducted by using red blood cells of New Zealand white rabbit. The sample was added with lyophilized powder 4 at a concentration of 5 mg/mL, and a negative control group (0.9% sodium chloride injection), an excipient control group (human serum albumin) and a positive control group (deionized water) were set up at the same time.

The results showed that, there was no hemolysis or aggregation of red blood cells in the negative control tube (0.9% sodium chloride injection), while hemolysis occurred in the positive control tube (deionized water), proving that this experimental system can accurately reflect the hemolytic effect of the test sample/control on red blood cells. During the experiment, there was no hemolysis or aggregation of red blood cells in the test sample tubes and excipient control tubes.

The results showed that the lyophilized powder 4 at a concentration of 5 mg/mL did not cause hemolysis or aggregation of red blood cells.

### Example 21. Vascular/Muscle Stimulation Experiment

In this experiment, 9 New Zealand white rabbits regardless of gender were selected and randomly divided into 3 groups, including an excipient control group (human serum albumin 22.8mg/kg, consistent with the protein content of the high-dose group of the test sample), 11mg/kg and 16.5mg/kg groups of lyophilized powder 4. The drug was administered by bolus injection into the ear vein in equal concentrations (5 mg/ml) and unequal volumes, once a week for a total of 2 times. On the last administration day of the vascular stimulation experiment, lyophilized powder 4 was injected into the hind limbs intramuscularly. The experiment used left and right self-consubstantiality control, and 0.9% sodium chloride injection was intravenously or intramuscularly injected.

The results showed that no vascular or muscle stimulation related to the test sample was observed in all animals by naked eye and histopathological observation.

### Example 22. Acute toxicity experiment in CD1 mice

In this experiment, 80 CD1 mice, half male and half female, were selected and randomly divided into 8 groups, including a negative control group (0.9% sodium chloride injection), an excipient control group (human serum albumin 103.5 mg/kg, consistent with the protein content of the high-dose group of the test sample), 25 mg/kg, 75 mg/kg and 225 mg/kg groups of the test sample lyophilized powder 4, and 25mg/kg, 75mg/kg and 225mg/kg groups of the commercial control Abraxane^{®}. The drug was administered 3 times by intravenous injection at equal concentrations (5 mg/ml) and unequal volumes, with an interval of at least 4 hours between each administration.

The results showed that no animal died/was dying in all groups. The Lyophilized powder 4 and Abraxane^{®} had similar toxicity profile, with a maximum tolerated dose (MTD) of 225mg/kg/day.

### Example 23 Preparation of docetaxel albumin nanoparticle for rapid suspension and subsequent property investigation

3g of docetaxel was dissolved in 15ml of methylene chloride/ethanol (1:1, v/v), and then added with 500ml of human serum albumin solution (4% w/v). The mixture was homogenated by a high-shear disperser (fluko FZ-20) for 2 minutes to form a primary emulsion, and then homogenized by a high-pressure homogenizer at 10,000-20,000 psi to obtain a nanoemulsion. The nanoemulsion was transferred to a rotary evaporator, heated in a water bath at 40 °C, and evaporated under a reduced pressure at 40 mbar to remove the organic solvent in the solution. The produced docetaxel albumin nanoparticles had a diameter of 102 nm, and the suspension was translucent.

The obtained docetaxel albumin nanoparticle suspension was dialyzed 5 folds by volume by a 100kD ultrafiltration membrane using a 0.9% sodium chloride solution. After dialysis, the average diameter of the nanoparticles was 104 nm, and there was no significant change in particle size during the dialysis. A docetaxel purified nanoparticle suspension was obtained as a white translucent liquid.

The purified docetaxel nanoparticle suspension after dialysis passed smoothly through a 0.22 micron filter membrane. The docetaxel content in the suspension was determined by HPLC, and the suspension was packaged by adjusting the package amount to 50 mg/vial according to the content, and lyophilized in a lyophilizer for 75 hours to obtain a stable white cake.

The white cake obtained after lyophilization was added with water for injection and shaken gently to be dissolved completely. The white cake was completely dissolved within 2 minutes, and the suspension was translucent with only slight foaming. The resuspended suspension did not have significant change in particle size, compared with that before lyophilization, and no significant changes were found in the appearance and transmittance after being left at room temperature for 24 hours. The contents of human serum albumin and docetaxel in the product were determined respectively, and the ratio of albumin to docetaxel was found to be 0.83:1.

### Example 24 Preparation of cabazitaxel albumin nanoparticle for rapid suspension and subsequent property investigation

500 mg of cabazitaxel was dissolved in 3 ml of chloroform/ethanol (8:1, v/v), and then added with 150 ml of human serum albumin solution (5% w/v). The mixture was homogenated by a high-shear disperser (fluko FZ-20) for 2 minutes to form a primary emulsion, and then homogenized by a high-pressure homogenizer at 10,000-20,000 psi to obtain a nanoemulsion. The nanoemulsion was transferred to a rotary evaporator, heated in a water bath at 40 °C, and evaporated under a reduced pressure at 40 mbar to remove the organic solvent in the solution. The produced cabazitaxel albumin nanoparticles had a diameter of 100nm, and the suspension was translucent.

The obtained cabazitaxel albumin nanoparticle suspension was added with an appropriate amount of sodium chloride so that the concentration of sodium chloride was 0.4%. A 100kD ultrafiltration membrane and a 5% mannitol + 1% sucrose solution were used for dialysis. The dialysis was 3 folds in volume. The nanoparticles had an average diameter of 101 nm, and there was no significant change in particle size during the dialysis. A purified cabazitaxel nanoparticle suspension was obtained as a white translucent liquid.

The purified cabazitaxel nanoparticle suspension after dialysis passed smoothly through a 0.22 micron filter membrane. The cabazitaxel content in the suspension was determined by HPLC. The suspension was packaged by adjusting the package amount to 5 mg/vial according to the content, and lyophilized in a lyophilizer for 48 hours to obtain a stable white cake.

The white cake obtained after lyophilization was added with water for injection and shaken gently to be dissolved completely. The white cake was completely dissolved within 2 minutes, and the suspension was translucent with only slight foaming. The resuspended suspension did not have significant change in particle size, compared with that before lyophilization, and no significant changes were found in the appearance and transmittance after being left at room temperature for 24 hours. The contents of human serum albumin and cabazitaxel in the product were determined respectively, and the ratio of albumin to cabazitaxel was found to be 2.53:1.

### Example 25 Preparation of docetaxel acetyl derivative albumin nanoparticle for rapid suspension and subsequent property investigation

200 mg of docetaxel acetyl derivative was dissolved in 2 ml of chloroform/ethanol (1:1, v/v), and then added with 100 ml of human serum albumin solution (5% w/v). The mixture was homogenated by a high-shear disperser (fluko FZ-20) for 2 minutes to form a primary emulsion, and then homogenized by a high-pressure homogenizer at 10,000-20,000 psi to obtain a nanoemulsion. The nanoemulsion was transferred to a rotary evaporator, heated in a water bath at 40 °C, and evaporated under a reduced pressure at 40 mbar to remove the organic solvent in the solution. The produced docetaxel acetyl derivative albumin nanoparticles had a diameter of 122 nm, and the suspension was translucent.

The obtained docetaxel acetyl derivative albumin nanoparticle suspension was dialyzed by a 1000kD ultrafiltration membrane with a 5% mannitol + 0.2% NaCl solution. The dialysis was 4 folds in volume. The nanoparticles had an average diameter of 161 nm, and there was no significant change in particle size during the dialysis. A purified docetaxel acetyl derivative nanoparticle suspension was obtained as a white translucent liquid.

The purified docetaxel acetyl derivative nanoparticle suspension after dialysis passed smoothly through a 0.22 micron filter membrane. The docetaxel acetyl derivative content in the suspension was determined by HPLC, and the suspension was packaged by adjusting the package amount to 5 mg/vial according to the content, and lyophilized in a lyophilizer for 48 hours to obtain a stable white cake.

The white cake obtained after lyophilization was added with water for injection and shaken gently to be dissolved completely. The white cake was completely dissolved within 2 minutes, and the suspension was translucent with only slight foaming. The resuspended suspension did not have significant change in particle size, compared with that before lyophilization, and no significant changes were found in the appearance and transmittance after being left at room temperature for 24 hours. The contents of human serum albumin and docetaxel acetyl derivative in the product were determined respectively, and the ratio of albumin to docetaxel acetyl derivative was found to be 1.66:1.

### Example 26 Preparation of rapamycin albumin nanoparticle for rapid suspension and subsequent property investigation

166 mg of rapamycin was dissolved in 1 ml of chloroform, and then added with 40 ml of human serum albumin solution (5% w/v). The mixture was homogenated by a high-shear disperser (fluko FZ-20) for 2 minutes to form a primary emulsion, and then homogenized by a high-pressure homogenizer at 10,000-20,000 psi to obtain a nanoemulsion. The nanoemulsion was transferred to a rotary evaporator, heated in a water bath at 40 °C, and evaporated under a reduced pressure at 40 mbar to remove the organic solvent in the solution. The produced rapamycin albumin nanoparticles had a diameter of 78 nm, and the suspension was translucent.

The obtained rapamycin albumin nanoparticle suspension was added with an appropriate amount of sodium chloride so that the concentration of sodium chloride was 0.9%. A 100kD ultrafiltration membrane and a 4% mannitol + 2% sucrose solution were used for dialysis. The dialysis was 4 folds in volume. The nanoparticles had an average diameter of 61 nm, and there was no significant change in particle size during the dialysis. A purified rapamycin nanoparticle suspension was obtained as a white translucent liquid.

The purified rapamycin nanoparticle suspension after dialysis passed smoothly through a 0.22 micron filter membrane. The rapamycin content in the suspension was determined by HPLC. The suspension was packaged by adjusting the package amount to 10 mg/vial according to the content, and lyophilized in a lyophilizer for 75 hours to obtain a stable white cake.

The white cake obtained after lyophilization was added with water for injection and shaken gently to be dissolved completely. The white cake was completely dissolved within 2 minutes, and the suspension was translucent with only slight foaming. The resuspended suspension did not have significant change in particle size, compared with that before lyophilization, and no significant changes were found in the appearance and transmittance after being left at room temperature for 24 hours. The contents of human serum albumin and rapamycin in the product were determined respectively, and the ratio of albumin to rapamycin was found to be 1.53:1.

### Example 27. Preparation of temsirolimus albumin nanoparticle for rapid suspension and subsequent property investigation

101 mg of temsirolimus was dissolved in 0.5 ml of dichloromethane, and then added with 19.5 ml of human serum albumin solution (5% w/v). The mixture was homogenated by a high-shear disperser (fluko FZ- 20) for 2 minutes to form a primary emulsion, and then homogenized by a high-pressure homogenizer at 10,000-20,000 psi to obtain a nanoemulsion. The nanoemulsion was transferred to a rotary evaporator, heated in a water bath at 40 °C, and evaporated under a reduced pressure at 40 mbar to remove the organic solvent in the solution. The produced temsirolimus albumin nanoparticles had a diameter of 88 nm, and the suspension was translucent.

The obtained temsirolimus albumin nanoparticle suspension was added with an appropriate amount of sodium chloride so that the concentration of sodium chloride was 1.0%. A 100kD ultrafiltration membrane and a 5% mannitol + 1% sucrose solution were used for dialysis. The dialysis was 3 folds in volume. The nanoparticles had an average diameter of 77 nm, and there was no significant change in particle size during the dialysis. A purified temsirolimus nanoparticle suspension was obtained as a white translucent liquid.

The purified temsirolimus nanoparticle suspension after dialysis passed smoothly through a 0.22 micron filter membrane. The temsirolimus content in the suspension was determined by HPLC. The suspension was packaged by adjusting the package amount to 10 mg/vial according to the content, and lyophilized in a lyophilizer for 66 hours to obtain a stable white cake.

The white cake obtained after lyophilization was added with 20 ml of water for injection and shaken gently to be dissolved completely. The white cake was completely dissolved within 2 minutes, and the suspension was translucent with only slightly foaming. The resuspended suspension did not have significant change in particle size, compared with that before lyophilization, and no significant changes were found in the appearance and transmittance after being left at room temperature for 24 hours. The contents of human serum albumin and temsirolimus in the product were determined respectively, and the ratio of albumin to temsirolimus was found to be 2.27:1.

### Example 28. Preparation of epothilone B albumin nanoparticle for rapid suspension and subsequent property investigation

127 mg of epothilone B was dissolved in 2 ml of chloroform/ethanol (10:1, v/v), and then added with 52 ml of human serum albumin solution (5% w/v). The mixture was homogenated by a high-shear disperser (fluko FZ-20) for 2 minutes to form a primary emulsion, and then homogenized by a high-pressure homogenizer at 10,000-20,000 psi to obtain a nanoemulsion. The nanoemulsion was transferred to a rotary evaporator, heated in a water bath at 40 °C, and evaporated under a reduced pressure at 40 mbar to remove the organic solvent in the solution. The produced epothilone B albumin nanoparticles had a diameter of 113 nm, and the suspension was translucent.

The obtained epothilone B albumin nanoparticle suspension was dialyzed by a 300kD ultrafiltration membrane with a 5% sucrose + 0.5% sodium chloride solution. The dialysis was 5 folds in volume. The nanoparticles had an average diameter of 102 nm, and there was no significant change in particle size during the dialysis. A purified epothilone B nanoparticle suspension was obtained as a white translucent liquid.

The purified epothilone B nanoparticle suspension after dialysis passed smoothly through a 0.22 micron filter membrane. The epothilone B content in the suspension was determined by HPLC, and the suspension was packaged by adjusting the package amount to 10 mg/vial according to the content, and lyophilized in a lyophilizer for 65 hours to obtain a stable white cake.

The white cake obtained after lyophilization was added with 20 ml of water for injection and shaken gently to be dissolved completely. The white cake was completely dissolved within 2 minutes, and the suspension was translucent with only slight foaming. The resuspended suspension did not have significant change in particle size, compared with that before lyophilization, and no significant changes were found in the appearance and transmittance after being left at room temperature for 24 hours. The contents of human serum albumin and epothilone B in the product were determined respectively, and the ratio of albumin to epothilone B was found to be 1.03:1.

### Example 29. Preparation of tanespimycin albumin nanoparticle for rapid suspension and subsequent property investigation

78 mg of tanespimycin was dissolved in 0.6 ml of chloroform, and then added with 22 ml of human serum albumin solution (8% w/v). The mixture was homogenated by a high-shear disperser (fluko FZ-20) for 2 minutes to form a primary emulsion, and then homogenized by a high-pressure homogenizer at 10,000-20,000 psi to obtain a nanoemulsion. The nanoemulsion was transferred to a rotary evaporator, heated in a water bath at 40 °C, and evaporated under a reduced pressure at 40 mbar to remove the organic solvent in the solution. The produced tanespimycin albumin nanoparticles had a diameter of 105nm, and the suspension was translucent.

The obtained tanespimycin albumin nanoparticle suspension was added with an appropriate amount of sodium chloride so that the concentration of sodium chloride was 0.9%. A 300kD ultrafiltration membrane and a 3% mannitol + 3% sucrose solution were used for dialysis. The dialysis was 4 folds in volume. The nanoparticles had an average diameter of 102 nm, and there was no significant change in particle size during the dialysis. A purified tanespimycin nanoparticle suspension was obtained as a purple liquid.

The purified tanespimycin nanoparticle suspension after dialysis passed smoothly through a 0.22 micron filter membrane. The tanespimycin content in the suspension was determined by HPLC. The suspension was packaged by adjusting the package amount to 10 mg/vial according to the content, and lyophilized in a lyophilizer for 45 hours to obtain a stable purple cake.

The purple cake obtained after lyophilization was added with water for injection and shaken gently to be dissolved completely. The purple cake was completely dissolved within 2 minutes, and the suspension was purple in color with only slight foaming. The resuspended suspension did not have significant change in particle size, compared with that before lyophilization, and no significant changes were found in the appearance and transmittance after being left at room temperature for 24 hours. The contents of human serum albumin and tanespimycin in the product were determined respectively, and the ratio of albumin to tanespimycin was found to be 1.76:1.

### Example 30. Preparation of 10-hydroxycamptothecin albumin nanoparticle for rapid suspension and subsequent property investigation

93 mg of 10-hydroxycamptothecin was dissolved in 2 ml of dichloromethane/ethanol (10:1, v/v), and then added with 48 ml of human serum albumin solution (5% w/v). The mixture was homogenated by a high-shear disperser (fluko FZ-20) for 2 minutes to form a primary emulsion, and then homogenized by a high-pressure homogenizer at 10,000-20,000 psi to obtain a nanoemulsion. The nanoemulsion was transferred to a rotary evaporator, heated in a water bath at 40 °C, and evaporated under a reduced pressure at 40 mbar to remove the organic solvent in the solution. The produced 10-hydroxycamptothecin albumin nanoparticles had a diameter of 118nm, and the suspension was yellow and translucent.

The obtained 10-hydroxycamptothecin albumin nanoparticle suspension was dialyzed by a 300kD ultrafiltration membrane with a 5% mannitol + 0.1% sodium chloride solution. The dialysis was 4 folds in volume. The nanoparticles had an average diameter of 102 nm, and there was no significant change in particle size during the dialysis. A purified 10-hydroxycamptothecin nanoparticle suspension was obtained as a yellow translucent liquid.

The purified 10-hydroxycamptothecin nanoparticle suspension after dialysis passed smoothly through a 0.22 micron filter membrane. The 10-hydroxycamptothecin content in the suspension was determined by HPLC, and the suspension was packaged by adjusting the package amount to 20 mg/vial according to the content, and lyophilized in a lyophilizer for 45 hours to obtain a stable yellow cake.

The yellow cake obtained after lyophilization was added with water for injection and shaken gently to be dissolved completely. The yellow cake was completely dissolved within 2 minutes, and the suspension is yellow and translucent with only slight foaming. The resuspended suspension did not have significant change in particle size, compared with that before lyophilization, and no significant changes were found in the appearance and transmittance after being left at room temperature for 24 hours. The contents of human serum albumin and 10-hydroxycamptothecin in the product were determined respectively, and the ratio of albumin to 10-hydroxycamptothecin was found to be 1.49:1.

## Claims

1. A nanoparticle composition for rapid suspension comprising an active ingredient, a protein, a particle stabilizer, and optionally a lyoprotectant.

2. The nanoparticle composition of claim 1, wherein the composition comprises an active ingredient, a protein, a lyoprotectant and a particle stabilizer.

3. The nanoparticle composition of claim 1 or 2, wherein the active ingredient is selected from active ingredients suitable for being wrapped in human serum albumin, and should have the following characteristics: insoluble or slightly soluble in water, and soluble or easily soluble in a specific organic solvent.

4. The nanoparticle composition of claim 3, wherein the active ingredient is selected from the group consisting of
taxanes, including but not limited to paclitaxel, docetaxel, cabazitaxel, and lipophilic derivatives of docetaxel;
macrolides, including but not limited to rapamycin and derivatives thereof, epothilone B and derivatives thereof, tanespimycin and derivatives thereof;
camptothecins, including but not limited to 10-hydroxycamptothecin, SN38 and derivatives thereof, etc.;
anthracyclines, including but not limited to aclarubicin, pirarubicin, etc.; and
other active ingredients, including colchicine and derivatives thereof, thiocolchicine dimer, amiodarone, liothyronine, cyclosporine, exemestane, flutamide, fulvestrant, romidepsin, semustine, ibuprofen, etc.,
preferably, the active ingredient is paclitaxel or docetaxel.

5. The nanoparticle composition of claim 1 or 2, wherein the protein is selected from serum albumins with a carrier function, such as human serum albumin and bovine serum albumin, and is preferably human serum albumin.

6. The nanoparticle composition of claim 1 or 2, wherein the lyoprotectant is selected from the group consisting of mannitol, sucrose, lactose, maltose, trehalose, dextran or any combination thereof, preferably is a combination of mannitol and sucrose, with the ratio therebetween preferably of 10:1 to 1:1, preferably 8:1 to 3:1, and most preferably 5:1.

7. The nanoparticle composition of claim 1 or 2, wherein the particle stabilizer is selected from the group consisting of sodium chloride, disodium hydrogen phosphate/sodium dihydrogen phosphate, and potassium chloride.

8. The nanoparticle composition of claim 7, wherein the particle stabilizer is sodium chloride.

9. The nanoparticle composition of claim 1 or 2, wherein the nanoparticle composition for rapid suspension comprises, by weight percentage, 1 to 30% of an active ingredient, 0.1 to 30% of a protein, 0.00001-3% of a particle stabilizer, and balance of lyoprotectant.

10. The nanoparticle composition of claim 9, wherein the nanoparticle composition for rapid suspension comprises, by weight percentage, 0.00001-3%, preferably 0.0001-1%, or any subrange within the range, such as 0.0001-1%, 0.0001-0.1%, 0.0005-0.5%, 0.001-0.1%, 0.005-0.05%, 0.008-0.022% of the particle stabilizer.

11. The nanoparticle composition of claim 9, wherein the nanoparticle composition for rapid suspension comprises, by weight percentage, 1-30%, preferably 2-20%, or any subrange within the range, such as 3-15%, 5-10% of the active ingredient.

12. The nanoparticle composition of claim 9, wherein the nanoparticle composition for rapid suspension comprises, by weight percentage, 0.1 to 30%, or any subrange within the range, such as 1-25%, 5-20% of human serum albumin.

13. The nanoparticle composition of claim 1 or 2, wherein the binding rate of the active ingredient to protein is great than 90%, preferably 94% or more.

14. The nanoparticle composition of claim 1 or 2, wherein in the composition, the weight ratio of protein to active ingredient is 0.1:1-5:1, for example, is selected from the group consisting of 0.1:1, 0.2:1, 0.3:1, 0.4:1, 0.5:1, 0.6:1, 0.70:1, 0.8:1, 0.9:1, 0.95:1, 1:1, 1.1:1, 1.2:1, 1.3:1, 1.4: 1. 1.5:1, 1.6:1, 1.7:1, 1.8:1, 1.8:1, 1.9:1, 2:1, 2.1:1, 2.2:1, 2.3:1, 2.4:1, 2.5:1, 2.6:1, 2.7:1, 2.8:1, 2.9:1, 3:1, 3.5:1, 4:1, 4.5:1, 5:1 or a range between any two of the above ratios.

15. The nanoparticle composition of claim 1 or 2, wherein the nanoparticle for rapid suspension in the composition has an average particle size of 30-200 nm, for example, selected from the group consisting of 30, 40, 50, 60, 70, 80, 90, 100, 110, 120, 121, 122, 123, 124, 125, 126, 127, 128, 129, 130, 131, 132, 133, 134, 135, 136, 137, 138, 140, 141, 142, 143, 144, 145, 146, 147, 148, 149, 150, 151, 152, 153, 154, 155, 156, 157, 158, 159, 160, 165, 170, 175, 180, 185, 190, 195, 200 nm or a range between any two of the above values.

16. The nanoparticle composition of claim 1 or 2, wherein the composition does not comprise any surfactant.

17. A pharmaceutical composition comprising the nanoparticle composition of claim 1 or 2, wherein the pharmaceutical composition is in a solid or liquid form.

18. The pharmaceutical composition of claim 17, wherein the composition is an injection, dry powder or lyophilized powder.

19. The pharmaceutical composition of claim 18, wherein when the pharmaceutical composition is provided in a liquid form, the nanoparticle for rapid suspension are suspended in a pharmaceutically acceptable carrier, including but not limited to a buffer, a preservative, water for injection, physiological saline and isotonic solution, and the pharmaceutical composition in the liquid form comprises the active ingredient in a content of 0.1-100 mg/ml, preferably 0.5-50 mg/ml, more preferably 1-20 mg/ml, such as 5 mg/ml.

20. The pharmaceutical composition of claim 18, wherein when the composition is provided in a solid form, the nanoparticle composition for rapid suspension comprises the active ingredient in a content of 1-30%, preferably 2-20% or 3 -15% or 5-10% by weight.

21. A preparation method of the nanoparticle composition for rapid suspension of any one of claims 1-16, comprising the steps of:
(1) dissolving the active ingredient in an organic solvent to form an organic phase, and dissolving the protein in water to form an aqueous phase,
(2) mixing and homogenizing the organic phase and the aqueous phase to form a nanoemulsion,
(3) removing the organic solvent in the nanoemulsion to obtain a suspension,
(4) dialyzing, and
optionally (5) lyophilizing.

22. The preparation method of claim 21, wherein the organic solvent is selected from the group consisting of pure solvents with low water solubility and low boiling point, or mixed solvents thereof with a small molecular alcohol, preferably, the organic solvent is one or more selected from the group consisting of trichloromethane, methylene chloride, ethanol or tert-butanol, preferably chloroform or a combination of chloroform and ethanol.

23. The preparation method of claim 21, wherein the dialyzing is performed by adding a particle stabilizer to the suspension obtained in step (3) so that the concentration of the particle stabilizer in the suspension is 0.01%-1.4% (w/v), and dialyzing in an aqueous solution of lyoprotectant, preferably, the concentration of the particle stabilizer in the suspension is 0.1%-1.4% (w/v), preferably 0.1%-0.9% (w/v), preferably, the aqueous solution of the lyoprotectant comprises 1%-10%, preferably 2%-8%, preferably 5%-6% of the lyoprotectant.

24. The preparation method of claim 21, wherein the dialyzing is performed by dialyzing in an aqueous solution of a lyoprotectant, and the aqueous solution further comprises a particle stabilizer in a concentration of 0.01%-1.4% (w/v), preferably 0.05%-0.9% (w/v), preferably 0.05%-0.5% (w/v), and more preferably 0.05%-0.15%, preferably, the aqueous solution of the lyoprotectant comprises 1%-10%, preferably 2%-8%, preferably 5%-6% of the lyoprotectant.

25. The preparation method of claim 21, wherein the dialyzing is performed by dialyzing in a solution of a particle stabilizer with an appropriate concentration of 0.01%-1.4% (w/v), preferably 0.1% -1.4% (w/v), preferably 0.1%-0.9% (w/v), and optionally, adding a lyoprotectant after the dialysis, preferably, adding the lyoprotectant until the resulting liquid comprises 1%-10%, preferably 2%-8%, more preferably 5%-6% of the lyoprotectant.

26. The preparation method of claim 21, wherein the lyophilizing in step (5) is performed by freezing between -20°C and -60°C and drying at a temperature between 0°C and +40°C.

27. The preparation method of claim 21, wherein the dialyzing is performed in a dialysis fold of 2-10, preferably 3-6.

28. The preparation method of any one of claims 24-25, wherein the particle stabilizer is sodium chloride.

29. An nanoparticle composition for rapid suspension comprising an active ingredient, a protein, a lyoprotectant and a particle stabilizer, wherein the composition is prepared by the method of any one of claims 21-28.

30. Use of the nanoparticle composition of claims 1-16 or 29 or the pharmaceutical composition of claims 17-20 in the preparation of a medicament for treating a disease responsive to the active ingredient.

31. The use of claim 30, wherein the disease is selected from cancers, preferably liver cancer, prostate cancer, lung cancer, breast cancer, multiple myeloma, transplant rejection, colon cancer and lymphoma.
